# EUROPEAN PATENT APPLICATION

(11) **EP 4 382 515 A1**
(43) Date of publication of application: **12.06.2024**
(21) Application number: 22852345.2
(22) Date of filing: 05.08.2022
(51) Int. Cl.: C07D 307/78, C07D 307/79, C07C 211/43, C07C 211/46, C07C 37/00, C07C 39/04, A61K 31/34, A61P 25/00

(54) **BENZO-RING-CONTAINING DERIVATIVE, AND PREPARATION METHOD THEREFOR AND USE THEREOF**

(30) Priority: 06.08.2021 CN 202110900267
(71) Applicant: Shujing Biopharma Co., Ltd, Shanghai 201208 (CN); Jiangsu NHWA Pharmaceutical Co., Ltd, Xuzhou, Jiangsu 221000 (CN)
(72) Inventor: FENG, Jiaquan, Shanghai 201208 (CN); ZOU, Yuanhai, Shanghai 201208 (CN); WAN, Zehong, Shanghai 201208 (CN)
(74) Representative: Lavoix
(86) International application number: PCT/CN2022/110558
(87) International publication number: WO 2023/011634

(57) **Abstract**

The present invention relates to a benzo-ring-containing derivative, and a preparation method therefor and the use thereof. Specifically, the present invention relates to a compound as represented by general formula (Ia), and a preparation method therefor, a pharmaceutical composition containing same, and the use thereof as a GABA receptor agonist in the field of the central nervous system, specifically in inducing and maintaining anesthesia of mammals.

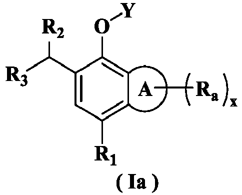

## Description

The present application claims priority to Chinese Patent Application No. 2021109002676 filed on Aug. 6, 2021, which is incorporated herein by reference in its entirety.

### TECHNICAL FIELD

The present invention belongs to the field of pharmaceutical chemistry, and particularly relates to a benzo-ring-containing derivative, a preparation method therefor, and use thereof.

### BACKGROUND

GABA_{A} receptors are the major inhibitory neurotransmitter receptors in the central nervous system. The GABA_{A} receptor is composed of a pentamer of transmembrane polypeptide subunits. 19 different subunits make up a variety of different GABA_{A} receptor subtypes. GABA_{A} receptors relate to the pathogenesis, diagnosis, and treatment of a variety of diseases including anesthesia, depression, anxiety, epilepsy, memory disorders, drug dependence, and the like. Therefore, the GABA_{A} receptor is an important drug target in pharmacology and clinical practice. Propofol and derivatives thereof are an important class of compounds targeting GABA_{A}.

Propofol can activate a plurality of GABA_{A} receptor subtypes as a short-acting intravenous general anesthetic, and has the advantages of quick response, no obvious accumulation, quick and complete revival. Propofol injection is clinically used for the induction and maintenance of general anesthesia. However, although propofol is a clinically mature intravenous anesthetic and is widely used, it has obvious limitations and disadvantages. Because of its poor water solubility, it is difficult to prepare suitable formulations, and clinically it has to be administered by injection in the form of an emulsion. Thus, the existing propofol medicament has the following defects: 1. the physical stability is poor; 2. vascular embolization may result from the larger oil droplet size; 3. about 70% of patients experience some degree of pain or discomfort from propofol injection; 4. it can be selectively mixed with only a few injectable products prior to administration; 5. the emulsion is easy to breed bacteria; 6. the toxic and side effects on the heart are easy to cause; 7. it can reduce systolic, diastolic and mean arterial blood pressure, and thus clinically cause hypotension; 8. adverse reactions such as respiratory depression are easy to cause. These disadvantages have largely limited the clinical application of propofol.

Therefore, based on the deficiencies in the prior art, there is an urgent need to develop a GABA_{A} receptor agonist drug with better stability, better pharmacodynamic properties and better safety, and less adverse drug reactions and side effects to meet the huge market demand.

### SUMMARY

For the technical problem to be solved, the present invention provides a benzo-ring-containing derivative, a preparation method therefor, and use thereof.

The present invention aims to provide a compound of general formula (Ia), a stereoisomer thereof, or a pharmaceutically acceptable salt thereof: 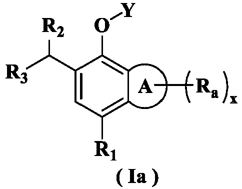 wherein:
ring A is C₅₋₆ cycloalkyl or 5- to 6-membered heterocyclyl;
Rₐ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl;
R₁ is hydrogen, deuterium, halogen, hydroxy, cyano, amino, C₁₋₆ alkoxy, C₁₋₆ alkyl, C₁₋₆ deuterated alkyl, or C₁₋₆ haloalkyl;
R₂ and R₃ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl;
Y is hydrogen, sodium, potassium, C₁₋₆ alkyl, or 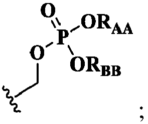
R_{AA} and R_{BB} are each independently hydrogen, deuterium, C₁₋₆ alkyl, C₁₋₆ deuterated alkyl, C₁₋₆ haloalkyl, an alkali metal ion, an alkaline earth metal ion, protonated amine, or protonated amino acid, wherein the alkali metal ion is Na⁺, K⁺, or Li⁺, the alkaline earth metal ion is Be²⁺, Mg²⁺, or Ca²⁺, the protonated amine is tromethamine, triethanolamine, ethanolamine, triethylamine, or N-methylglucamine, and the amino acid is arginine or lysine; and
x is an integer from 0 to 5.

In a certain preferred embodiment, ring A is C₅₋₆ cycloalkyl or 5- to 6-membered heterocyclyl containing 1 to 3 N, O, or S atoms.

In a certain preferred embodiment, ring A is preferably 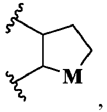 wherein:
M is CRₐₐR_{bb}, NRₐₐ, O, or S, preferably CRₐₐR_{bb} or O, more preferably CH₂ or O;
Rₐₐ and R_{bb} are each independently hydrogen, deuterium, halogen, hydroxy, cyano, amino, C₁₋₆ alkoxy, C₁₋₆ alkyl, C₁₋₆ deuterated alkyl, or C₁₋₆ haloalkyl, preferably hydrogen or C₁₋₆ alkyl, more preferably hydrogen or C₁₋₃ alkyl.

In a certain preferred embodiment, Rₐ are each independently hydrogen, C₁₋₆ alkyl, or C₃₋₆ cycloalkyl, preferably hydrogen, C₁₋₃ alkyl, or C₃₋₅ cycloalkyl, more preferably hydrogen, methyl, ethyl, propyl, or cyclopropyl, further preferably hydrogen, methyl, ethyl, or cyclopropyl. In a certain preferred embodiment, R₁ is hydrogen or halogen, preferably hydrogen, fluorine, chlorine, or bromine, more preferably hydrogen, fluorine, or chlorine.

In a certain preferred embodiment, R₂ and R₃ are each independently hydrogen, C₁₋₆ alkyl, or C₃₋₆ cycloalkyl, preferably hydrogen, C₁₋₃ alkyl, or C₃₋₅ cycloalkyl, more preferably hydrogen, methyl, ethyl, propyl, or cyclopropyl, further preferably methyl or cyclopropyl.

In a certain preferred embodiment, Y is hydrogen or

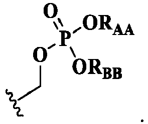

In a certain preferred embodiment, R_{AA} and R_{BB} are each independently hydrogen, C₁₋₆ alkyl, an alkali metal ion, or an alkaline earth metal ion, wherein the alkali metal ion is Na⁺, K⁺, or Li⁺, and the alkaline earth metal ion is Be²⁺, Mg²⁺, or Ca²⁺. R_{AA} and R_{BB} are preferably alkali metal ions, more preferably Na⁺.

In a certain preferred embodiment, x is 0, 1, or 2.

In a certain preferred embodiment, 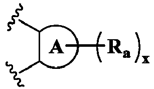 is

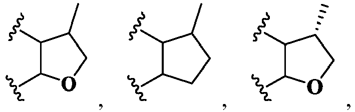

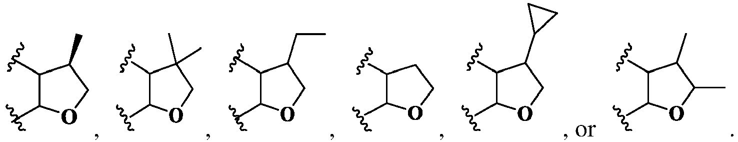

The present invention further provides a compound of general formula (I), a stereoisomer thereof, or a pharmaceutically acceptable salt thereof: wherein: 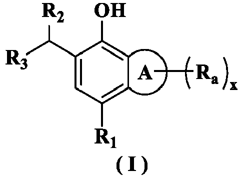
ring A is C₅₋₆ cycloalkyl or 5- to 6-membered heterocyclyl;
Rₐ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl;
R₁ is hydrogen, deuterium, halogen, hydroxy, cyano, amino, C₁₋₆ alkoxy, C₁₋₆ alkyl, C₁₋₆ deuterated alkyl, or C₁₋₆ haloalkyl;
R₂ and R₃ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl; and
x is an integer from 0 to 5.

In a further preferred embodiment of the present invention, ring A is C₅₋₆ cycloalkyl or 5- to 6-membered heterocyclyl containing 1 to 3 N, O, or S atoms.

In a further preferred embodiment of the present invention, ring A is preferably 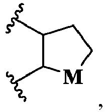 wherein:
M is CRₐₐR_{bb}, NRₐₐ, O, or S, preferably CRₐₐR_{bb} or O, more preferably CH₂ or O;
Rₐₐ and R_{bb} are each independently hydrogen, deuterium, halogen, hydroxy, cyano, amino, C₁₋₆ alkoxy, C₁₋₆ alkyl, C₁₋₆ deuterated alkyl, or C₁₋₆ haloalkyl, preferably hydrogen or C₁₋₆ alkyl, more preferably hydrogen or C₁₋₃ alkyl.

In a further preferred embodiment of the present invention, Rₐ are each independently hydrogen, C₁₋₆ alkyl, or C₃₋₆ cycloalkyl, preferably hydrogen, C₁₋₃ alkyl, or C₃₋₅ cycloalkyl, more preferably hydrogen, methyl, ethyl, propyl, or cyclopropyl, further preferably hydrogen, methyl, ethyl, or cyclopropyl.

In a further preferred embodiment of the present invention, R₁ is hydrogen or halogen, preferably hydrogen, fluorine, chlorine, or bromine, more preferably hydrogen, fluorine, or chlorine.

In a further preferred embodiment of the present invention, R₂ and R₃ are each independently hydrogen, C₁₋₆ alkyl, or C₃₋₆ cycloalkyl, preferably hydrogen, C₁₋₃ alkyl, or C₃₋₅ cycloalkyl, more preferably hydrogen, methyl, ethyl, propyl, or cyclopropyl, further preferably methyl or cyclopropyl.

In a further preferred embodiment of the present invention, x is 0, 1, or 2.

In a further preferred embodiment of the present invention, 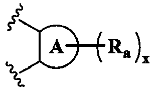 is

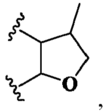

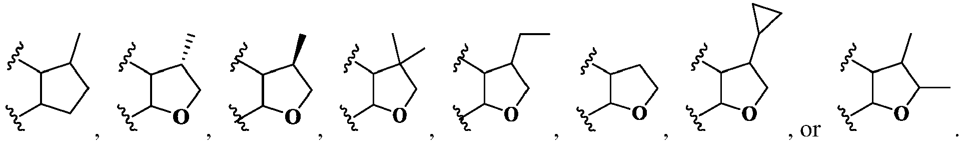

In a further preferred embodiment of the present invention, general formula (Ia) is further represented by general formula (IIa): 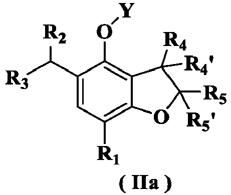 wherein:
R₄, R₄', R₅, and R₅' are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl, preferably hydrogen, C₁₋₆ alkyl, or C₃₋₆ cycloalkyl, more preferably hydrogen, C₁₋₃ alkyl, or C₃₋₅ cycloalkyl, further preferably hydrogen, methyl, ethyl, propyl, or cyclopropyl, particularly preferably hydrogen, methyl, ethyl, or cyclopropyl;
R₁, R₂, R₃, and Y are each as defined above in general formula (Ia).

In a certain preferred embodiment, R₄ and R₄' are each independently hydrogen, methyl, ethyl, or cyclopropyl.

In a certain preferred embodiment, R₅ and R₅' are each independently hydrogen or methyl.

In a further preferred embodiment of the present invention, general formula (I) is further represented by general formula (II): 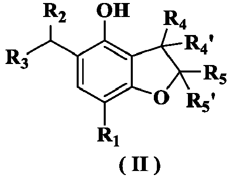 wherein:
R₄, R₄', R₅, and R₅' are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl, preferably hydrogen, C₁₋₆ alkyl, or C₃₋₆ cycloalkyl, more preferably hydrogen, C₁₋₃ alkyl, or C₃₋₅ cycloalkyl, further preferably hydrogen, methyl, ethyl, propyl, or cyclopropyl, particularly preferably hydrogen, methyl, ethyl, or cyclopropyl;
R₁, R₂, and R₃ are each as defined above in general formula (I).

In a certain preferred embodiment, R₄ and R₄' are each independently hydrogen, methyl, ethyl, or cyclopropyl.

In a certain preferred embodiment, R₅ and R₅' are each independently hydrogen or methyl.

In a further preferred embodiment of the present invention, R₁ is hydrogen or halogen, preferably hydrogen, fluorine, chlorine, or bromine, further preferably hydrogen, fluorine, or chlorine.

In a further preferred embodiment of the present invention, R₂ and R₃ are each independently hydrogen, C₁₋₆ alkyl, or C₃₋₆ cycloalkyl, preferably hydrogen, C₁₋₃ alkyl, or C₃₋₅ cycloalkyl, further preferably hydrogen, methyl, ethyl, propyl, or cyclopropyl, particularly preferably methyl or cyclopropyl.

In a further preferred embodiment of the present invention, the compounds of general formula (Ia), general formula (I), general formula (IIa), or general formula (II) described above do not include the following compounds: 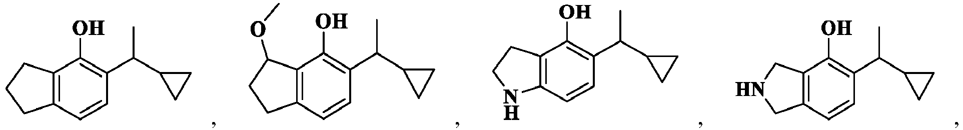 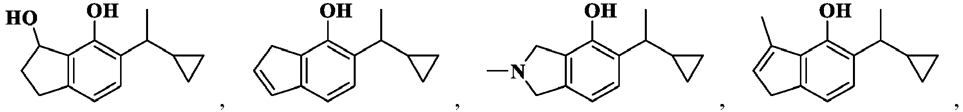 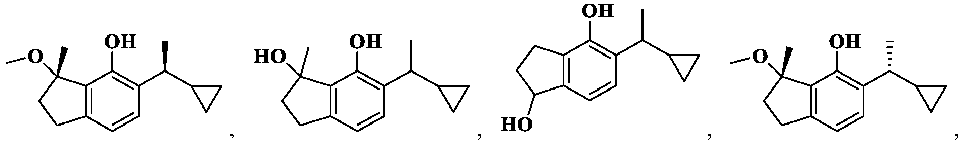 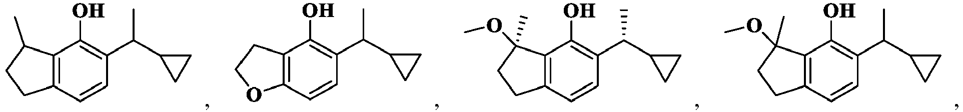 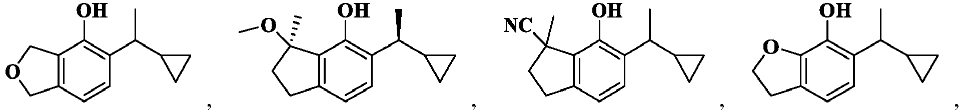 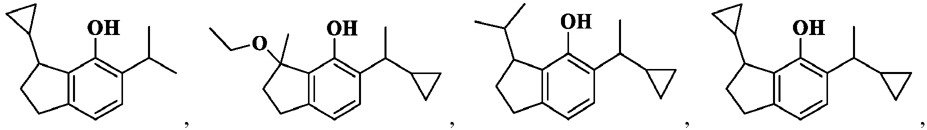 or

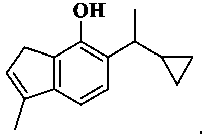

In a further preferred embodiment of the present invention, the compound is any one of the following structures:

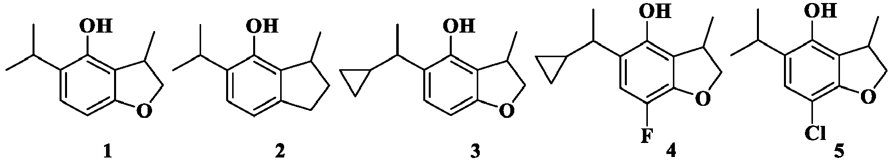

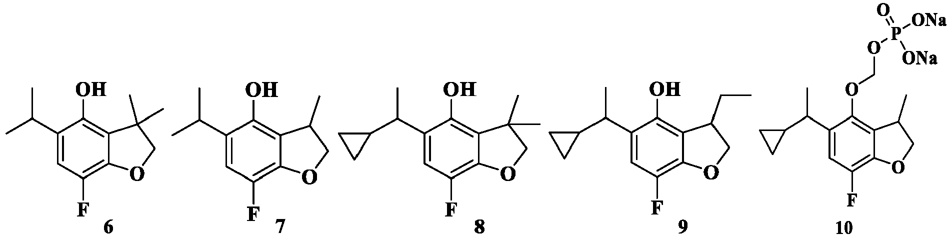

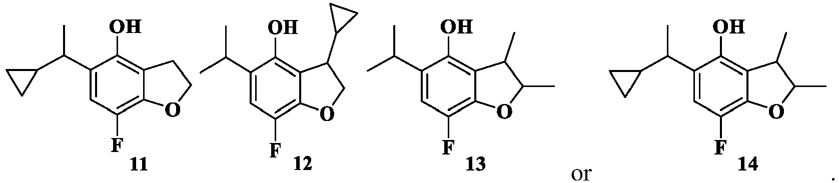

In a certain embodiment, the compound may be any one of the following structures: 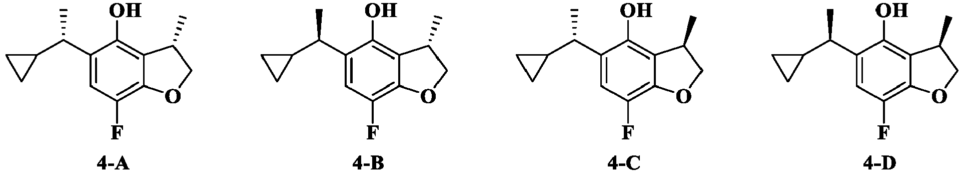 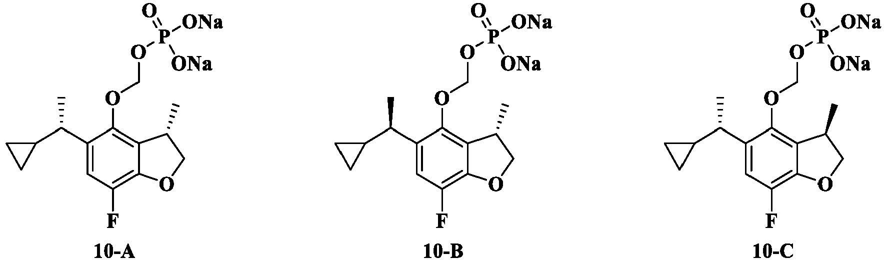 or

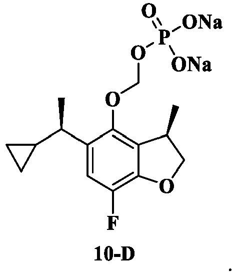

The present invention further provides a compound, wherein the compound is any one of the following compounds (-OBn is -oxybenzyl):

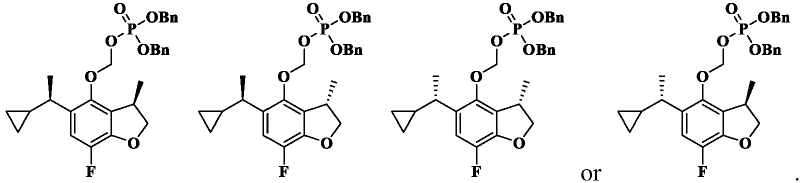

The present invention further provides a compound, wherein the compound is any one of the following compounds:
(1) a compound having a retention time of 5.976 min 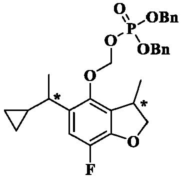 (chiral intermediate A1 for short) or a compound having a retention time of 9.428 min 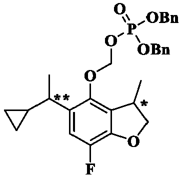 (chiral intermediate A2 for short) under the following conditions: chromatographic column: DAICEL CHIRALPAK^{®}IG, 250 × 25 mm 10 µm; mobile phase: n-hexane/ethanol = 80/20; flow rate: 30 mL/min; detection wavelength: 254 nm;
(2) a compound having a retention time of 1.956 min 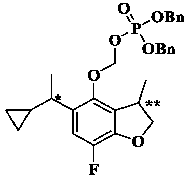 (chiral intermediate B1 for short) or a compound having a retention time of 2.224 min 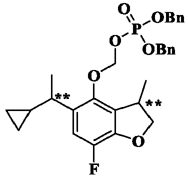 (chiral intermediate B2 for short) under the following conditions: chromatographic column: DAICEL CHIRALCEL^{®}OJ, 250 × 25 mm 10 µm; mobile phase: supercritical CO₂/MeOH (0.1% 7.0 mol/L ammonia in MeOH) = 80/20; flow rate: 120 mL/min; detection wavelength: 214 nm; the carbon atom with "*" is a chiral carbon atom present in a form of a single (R) or (S) enantiomer.

In a certain embodiment, compound 10A1 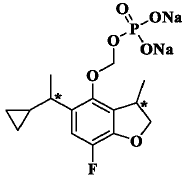 is prepared from the chiral intermediate A1 described above, Pd/C, and Na₂CO₃ via a debenzylation reaction, wherein the carbon atom with "*" is a chiral carbon atom present in a form of a single (R) or (S) enantiomer.

In a certain embodiment, compound 10A2 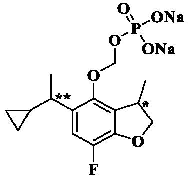 is prepared from the chiral intermediate A2 described above, Pd/C, and Na₂CO₃ via a debenzylation reaction, wherein the carbon atom with "*" is a chiral carbon atom present in a form of a single (R) or (S) enantiomer.

In a certain embodiment, compound 10B 1 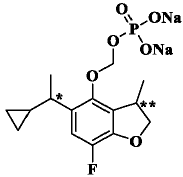 is prepared from the chiral intermediate B1 described above, Pd/C, and Na₂CO₃ via a debenzylation reaction, wherein the carbon atom with "*" is a chiral carbon atom present in a form of a single (R) or (S) enantiomer.

In a certain embodiment, compound 10B2 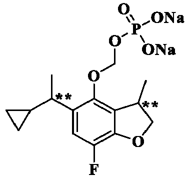 is prepared from the chiral intermediate B2 described above, Pd/C, and Na₂CO₃ via a debenzylation reaction, wherein the carbon atom with "*" is a chiral carbon atom present in a form of a single (R) or (S) enantiomer.

In a certain embodiment, compound 11A1 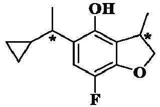 is prepared from the chiral intermediate A1 described above and hydrochloric acid via a protonation reaction, wherein the carbon atom with "*" is a chiral carbon atom present in a form of a single (R) or (S) enantiomer.

In a certain embodiment, compound 11A2 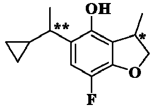 is prepared from the chiral intermediate A2 described above and hydrochloric acid via a protonation reaction, wherein the carbon atom with "*" is a chiral carbon atom present in a form of a single (R) or (S) enantiomer.

In a certain embodiment, compound 11B1 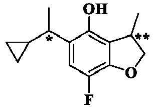 is prepared from the chiral intermediate B1 described above and hydrochloric acid via a protonation reaction, wherein the carbon atom with "*" is a chiral carbon atom present in a form of a single (R) or (S) enantiomer.

In a certain embodiment, compound 11B2 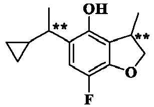 is prepared from the chiral intermediate B2 described above and hydrochloric acid via a protonation reaction, wherein the carbon atom with "*" is a chiral carbon atom present in a form of a single (R) or (S) enantiomer.

The present invention further provides a method for preparing a compound of general formula (I), a stereoisomer thereof, or a pharmaceutically acceptable salt thereof. A commercially available starting material may be used in the preparation method, which is synthesized according to a known method.

In a certain embodiment, the preparation method preferably comprises the following steps: in a solvent, performing a dehydroxylation reaction on a compound represented by formula 1-1 in the presence of a dehydroxylating reagent and an acid to give a compound represented by formula I; 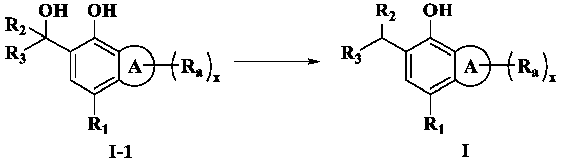 wherein ring A, R₁, R₂ and R₃, Rₐ, and x are as defined above.

In a certain embodiment, the conditions of the dehydroxylation reaction may be conventional conditions in the art for such reactions.

In a certain embodiment, the solvent may be a conventional solvent in the art for such reactions, such as a halogenated hydrocarbon solvent, preferably dichloromethane.

In a certain embodiment, the dehydroxylating reagent may be a conventional dehydroxylating reagent in the art for such reactions, such as a silane dehydroxylating reagent, preferably triethylsilane.

In a certain embodiment, the acid may be a conventional acid in the art for such reactions, such as a strong organic acid, preferably trifluoroacetic acid.

In a certain embodiment, the preparation method may further comprise the following steps: in a solvent, performing a substitution reaction on a compound represented by formula I-2 in the presence of a Grignard reagent R₃-MgX₁ to give a compound represented by formula 1-1; 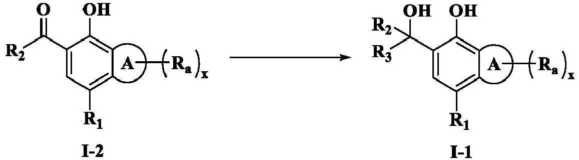 wherein R₃ is as defined above, and X₁ is halogen.

In a certain embodiment, the conditions of the substitution reaction may be conventional conditions in the art for such reactions.

In a certain embodiment, the halogen may be a conventional halogen in a Grignard reagent, such as Cl, Br, or I, preferably Br.

In a certain embodiment, the solvent may be a conventional solvent in the art for such reactions, such as an ether solvent, preferably tetrahydrofuran.

The present invention further provides a method for preparing a compound of general formula (Ia), a stereoisomer thereof, or a pharmaceutically acceptable salt thereof. A commercially available starting material may be used in the preparation method, which is synthesized according to a known method.

In a certain embodiment, the preparation method is preferably any one of the following methods: method I comprises the following step: in a solvent, performing a dehydroxylation reaction on a compound represented by formula Ia-1 in the presence of a dehydroxylating reagent and an acid to give a compound represented by formula Ia; 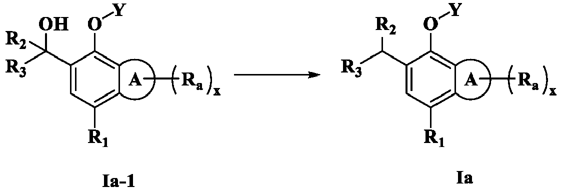
wherein ring A, R₁, R₂ and R₃, Rₐ, Y, and x are as defined above;
method II comprises the following step: in a solvent, performing a substitution reaction on a compound represented by formula I in the presence of X₂-Y and an alkali to give a compound represented by formula Ia; 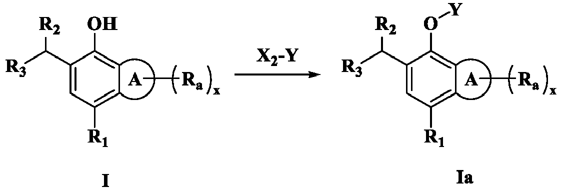
wherein ring A, R₁, R₂ and R₃, Rₐ, Y, and x are as defined above, and the definition of Y does not include H; X₂ is halogen.

In a certain embodiment, in method I, the conditions of the dehydroxylation reaction may be conventional conditions in the art for such reactions.

In a certain embodiment, in method I, the solvent may be a conventional solvent in the art for such reactions, such as a halogenated hydrocarbon solvent, preferably dichloromethane.

In a certain embodiment, in method I, the dehydroxylating reagent may be a conventional dehydroxylating reagent in the art for such reactions, such as a silane dehydroxylating reagent, preferably triethylsilane.

In a certain embodiment, in method I, the acid may be a conventional acid in the art for such reactions, such as a strong organic acid, preferably trifluoroacetic acid.

In a certain embodiment, in method II, the conditions of the substitution reaction may be conventional conditions in the art for such reactions.

In a certain embodiment, in method II, the halogen may be a conventional halogen in the art for such reactions, such as F, Cl, Br, or I, preferably Cl.

In a certain embodiment, in method II, the solvent may be a conventional solvent in the art for such reactions, such as an amide solvent, preferably *N,N*-dimethylformamide.

In a certain embodiment, in method II, the alkali may be a conventional alkali in the art for such reactions, such as an alkali metal hydride, preferably NaH.

In a certain embodiment, the method may further comprise the following step: in a solvent, performing a substitution reaction on a compound represented by formula Ia-2 in the presence of a Grignard reagent R₃-MgX₁ to give a compound represented by formula Ia-1; 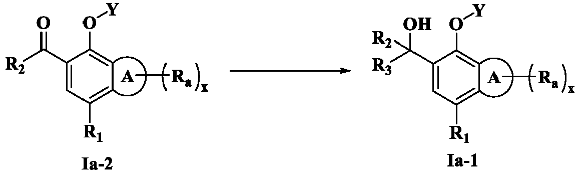 wherein R₃ is as defined above, and X₁ is halogen.

In a certain embodiment, the conditions of the substitution reaction may be conventional conditions in the art for such reactions.

In a certain embodiment, the halogen may be a conventional halogen in a Grignard reagent, such as Cl, Br, or I, preferably Br.

In a certain embodiment, the solvent may be a conventional solvent in the art for such reactions, such as an ether solvent, preferably tetrahydrofuran.

The present invention further relates to a pharmaceutical composition comprising a therapeutically effective dose of any one of the compounds, the stereoisomers thereof, or the pharmaceutically acceptable salts thereof, and one or more pharmaceutically acceptable carriers or excipients.

The present invention further relates to use of any one of the compounds, the stereoisomers thereof, or the pharmaceutically acceptable salts thereof, or the pharmaceutical composition thereof in the preparation of a GABA_{A} receptor agonist drug.

The present invention further relates to use of any one of the compounds, the stereoisomers thereof, or the pharmaceutically acceptable salts thereof, or the pharmaceutical composition thereof in the preparation of a drug in the central nervous system field.

In a further preferred embodiment of the present invention, the drug in the central nervous system field is a drug for inducing and maintaining anesthesia in a mammal, promoting sedation and hypnosis in a mammal, and treating and/or preventing anxiety, depression, insomnia, nausea, vomiting, migraine, schizophrenia, convulsions, or epilepsy.

The present invention further relates to use of any one of the compounds, the stereoisomers thereof, or the pharmaceutically acceptable salts thereof, or the pharmaceutical composition thereof in the preparation of a drug for inducing and maintaining anesthesia in a mammal, promoting sedation and hypnosis in a mammal, and treating and/or preventing anxiety, depression, insomnia, nausea, vomiting, migraine, schizophrenia, convulsions, or epilepsy.

The present invention further relates to a method for inducing and maintaining anesthesia in a mammal, promoting sedation and hypnosis in a mammal, and treating and/or preventing anxiety, depression, insomnia, nausea, vomiting, migraine, schizophrenia, convulsions, epilepsy or the like, comprising administering a therapeutically effective dose of any one of the compounds, the stereoisomers thereof, or the pharmaceutically acceptable salts thereof, or the pharmaceutical composition thereof to the mammal.

### Detailed Description of the Invention

Unless otherwise defined, all technical and scientific terms used herein have the same meaning as that commonly understood by those of ordinary skill in the art to which the present invention belongs. In the event of a contradiction, the definition provided in the present application will control. When referring to a trade name, it is intended to refer to its corresponding commercial product or its active ingredient. All patents, published patent applications and publications cited herein are incorporated herein by reference.

The term "alkyl" refers to a linear or branched saturated aliphatic hydrocarbon group consisting of carbon atoms and hydrogen atoms, which is linked to the rest of the molecule via a single bond. The "alkyl" may have 1-8 or more carbon atoms, i.e., "C₁-C₈ alkyl", e.g., C₁₋₄ alkyl, C₁₋₃ alkyl, C₁₋₂ alkyl, C₃ alkyl, C₄ alkyl, C₁₋₆ alkyl, or C₃₋₆ alkyl. Non-limiting examples of the alkyl include, but are not limited to, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof. The alkyl may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the alkyl is substituted with a substituent, the substituent is not further substituted.

The term "cycloalkyl" refers to a saturated or partially unsaturated monocyclic or polycyclic hydrocarbon substituent. The cycloalkyl contains 3 to 20 carbon atoms, preferably 3 to 12 carbon atoms, more preferably 3 to 6 carbon atoms, and most preferably 3 to 5 carbon atoms, or 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl includes spiro cycloalkyl, fused cycloalkyl, and bridged cycloalkyl.

The term "spiro cycloalkyl" refers to a 5- to 20-membered polycyclic group in which monocyclic rings share one carbon atom (referred to as the spiro atom). It may contain one or more double bonds, but none of the rings has a fully conjugated π-electron system. It is preferably 6- to 14-membered, and is more preferably 7- to 10-membered. According to the number of spiro atoms shared among the rings, the spiro cycloalkyl may be monospiro cycloalkyl, bispiro cycloalkyl, or polyspiro cycloalkyl, preferably monospiro cycloalkyl or bispiro cycloalkyl, and more preferably 3-membered/6-membered, 3-membered/5-membered, 4-membered/4-membered, 4-membered/5-membered, 4-membered/6-membered, 5-membered/5-membered, or 5-membered/6-membered monospiro cycloalkyl. Spiro heterocycloalkyl in which monospiro cycloalkyl shares a spiro atom with heterocycloalkyl is also included.

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of the rings has a fully conjugated π-electron system. It is preferably 6- to 14-membered, and is more preferably 7- to 10-membered. According to the number of constituent rings, the fused cycloalkyl may be bicyclic, tricyclic, tetracyclic, or polycyclic fused cycloalkyl, preferably bicyclic or tricyclic fused cycloalkyl, and more preferably 5-membered/5-membered or 5-membered/6-membered bicycloalkyl.

The term "bridged cycloalkyl" refers to a 5- to 20-membered all-carbon polycyclic group in which any two rings share two carbon atoms that are not directly linked to each other, wherein these rings contain one or more double bonds, but none of the rings has a fully conjugated π-electron system. It is preferably 6- to 14-membered, and is more preferably 7- to 10-membered. According to the number of constituent rings, the bridged cycloalkyl may be bicyclic, tricyclic, tetracyclic, or polycyclic bridged cycloalkyl, preferably bicyclic, tricyclic, or tetracyclic bridged cycloalkyl, and more preferably bicyclic or tricyclic bridged cycloalkyl.

The cycloalkyl described above can all be fused to an aryl, heteroaryl, or heterocycloalkyl ring, wherein the ring connected to the parent structure is the cycloalkyl. The cycloalkyl may be optionally substituted or unsubstituted. When it is substituted, the substituent may be substituted at any available linking site, and the substituent is preferably one or more of the following groups independently selected from alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the cycloalkyl is substituted with a substituent, the substituent is not further substituted.

The term "heterocyclyl" refers to a saturated or unsaturated monocyclic or polycyclic hydrocarbon substituent containing 3 to 20 ring atoms, wherein one or more of the ring atoms are heteroatoms selected from nitrogen, oxygen, and S(O)ₘ (wherein m is an integer from 0 to 2), excluding a cyclic portion of -O-O-, -O-S-, or -S-S-, and the remaining ring atoms are carbon atoms. Preferably, the heterocyclyl contains 3 to 12 ring atoms, of which 1 to 4 are heteroatoms; more preferably, the heterocyclyl contains 3 to 6 ring atoms, of which 1 to 3 are heteroatoms; further preferably, the heterocyclyl is 5- to 6-membered heterocyclyl containing 1 to 3 atoms selected from N, O, and S.

Non-limiting examples of monocyclic heterocyclyl include oxetanyl, thietanyl, pyrrolidinyl, imidazolidinyl, tetrahydrofuranyl, tetrahydrothienyl, tetrahydropyranyl, dihydroimidazolyl, dihydrofuranyl, dihydropyrazolyl, piperidinyl, piperazinyl, morpholinyl, 1,3-dioxolanyl, 2,2-difluoro-1,3-dioxolanyl, azepinyl, or the like. Non-limiting examples of polycyclic heterocyclyl include spiro heterocyclyl, fused heterocyclyl, and bridged heterocyclyl, wherein the spiro heterocyclyl, fused heterocyclyl, and bridged heterocyclyl involved are optionally linked to other groups via single bonds, or are further ortho-fused to other cycloalkyl, heterocyclyl, aryl, and heteroaryl via any two or more atoms of the ring.

The term "spiro heterocyclyl" refers to a 5- to 20-membered polycyclic heterocyclyl group in which monocyclic rings share one atom (referred to as the spiro atom), wherein one or more of the ring atoms are heteroatoms selected from nitrogen, oxygen, and S(O)ₘ (wherein m is an integer from 0 to 2), and the remaining ring atoms are carbon atoms. It may contain one or more double bonds, but none of the rings has a fully conjugated π-electron system. It is preferably 6-to 14-membered, and is more preferably 7- to 10-membered. According to the number of spiro atoms shared among the rings, the spiro heterocyclyl may be monospiro heterocyclyl, bispiro heterocyclyl, or polyspiro heterocyclyl, preferably monospiro heterocyclyl or bispiro heterocyclyl, and more preferably 3-membered/6-membered, 3-membered/5-membered, 4-membered/4-membered, 4-membered/5-membered, 4-membered/6-membered, 5-membered/5-membered, or 5-membered/6-membered monospiro heterocyclyl.

The term "fused heterocyclyl" refers to a 5- to 20-membered polycyclic heterocyclyl group in which each ring in the system shares a pair of adjacent atoms with the other rings in the system, wherein one or more of the ring atoms are heteroatoms selected from nitrogen, oxygen, or S(O)ₘ (wherein m is an integer from 0 to 2), and the remaining ring atoms are carbon atoms, wherein one or more of the rings may contain one or more double bonds, but none of the rings has a fully conjugated π-electron system. It is preferably 6- to 14-membered, and is more preferably 7- to 10-membered. According to the number of constituent rings, the fused heterocyclyl may be bicyclic, tricyclic, tetracyclic, or polycyclic fused heterocyclyl, preferably bicyclic or tricyclic fused heterocyclyl, and more preferably 5-membered/5-membered or 5-membered/6-membered bicyclic fused heterocyclyl.

The term "bridged heterocyclyl" refers to a 5- to 20-membered polycyclic heterocyclyl group in which any two rings share two atoms that are not directly linked to each other, wherein one or more of the ring atoms are heteroatoms selected from nitrogen, oxygen, or S(O)ₘ (wherein m is an integer from 0 to 2), and the remaining ring atoms are carbon atoms, wherein these rings contain one or more double bonds, but none of the rings has a fully conjugated π-electron system. It is preferably 6- to 14-membered, and is more preferably 7- to 10-membered. According to the number of constituent rings, the bridged heterocyclyl may be bicyclic, tricyclic, tetracyclic, or polycyclic bridged heterocyclyl, preferably bicyclic, tricyclic, or tetracyclic bridged heterocyclyl, and more preferably bicyclic or tricyclic bridged heterocyclyl.

The heterocyclyl described above all can be fused to an aryl, heteroaryl, or cycloalkyl ring, wherein the ring connected to the parent structure is the heterocyclyl. The heterocyclyl may be optionally substituted or unsubstituted. When it is substituted, the substituent may be substituted at any available linking site, and the substituent is preferably one or more of the following groups independently being alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the heterocyclyl is substituted with a substituent, the substituent is not further substituted.

The term "aryl" refers to a 6- to 14-membered, preferably 6- to 12-membered all-carbon monocyclic or fused polycyclic group having a conjugated π-electron system, such as phenyl and naphthyl, more preferably, phenyl. The aryl can be fused to a heteroaryl, heterocyclyl, or cycloalkyl ring, wherein the ring connected to the parent structure is the aryl ring, including benzo 5- to 10-membered heteroaryl, benzo 3- to 8-membered cycloalkyl, and benzo 3- to 8-membered heterocyclyl, preferably benzo 5- to 6-membered heteroaryl, benzo 3- to 6-membered cycloalkyl, and benzo 3- to 6-membered heterocyclyl. The aryl may be optionally substituted or unsubstituted. When it is substituted, the substituent may be substituted at any available linking site, and the substituent is preferably one or more of the following groups independently being alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the aryl is substituted with a substituent, the substituent is not further substituted.

The term "heteroaryl" refers to a heteroaromatic system containing 1 to 4 heteroatoms and 5 to 14 ring atoms, wherein the heteroatoms are selected from oxygen, sulfur, nitrogen, and the like. The heteroaryl is preferably 5- to 12-membered, and more preferably 5- to 6-membered, such as pyrrolyl, imidazolyl, furanyl, pyranyl, thienyl, thiazolyl, thiadiazolyl, pyrazolyl, oxazolyl, oxadiazolyl, triazolyl, tetrazolyl, pyridinyl, pyrimidinyl, pyridazinyl and pyrazinyl. The heteroaryl can be fused to an aryl, cycloalkyl, or heterocyclyl ring, wherein the ring connected to the parent structure is the heteroaryl ring. The heteroaryl may be optionally substituted or unsubstituted. When it is substituted, the substituent may be substituted at any available linking site, and the substituent is preferably one or more of the following groups independently being alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the heteroaryl is substituted with a substituent, the substituent is not further substituted.

The term "alkoxy" refers to -O-(alkyl), wherein the alkyl is as defined above. Non-limiting examples of alkoxy include: methoxy, ethoxy, propoxy, butoxy, and the like. The alkoxy may be optionally substituted or unsubstituted. When it is substituted, the substituent may be substituted at any available linking site, and the substituent is preferably one or more of the following groups independently being alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the alkoxy is substituted with a substituent, the substituent is not further substituted.

The term "alkylthio" refers to -S-(alkyl), wherein the alkyl is as defined above. Non-limiting examples of alkylthio include: methylthio, ethylthio, propylthio, butylthio, and the like. The alkylthio may be optionally substituted or unsubstituted. When it is substituted, the substituent may be substituted at any available linking site, and the substituent is preferably one or more of the following groups independently being alkyl, alkenyl, alkynyl, alkoxy, alkylthio, halogen, sulfydryl, hydroxy, nitro, amino, cyano, carboxyl, oxo, cycloalkyl, heterocyclyl, aryl, or heteroaryl. When the alkylthio is substituted with a substituent, the substituent is not further substituted.

The term "halo", "halogen", or "halogenated" should be understood to refer to fluorine (F), chlorine (Cl), bromine (Br), or iodine (I) atom, preferably fluorine, chlorine, or bromine atom.

The term "deuterated alkyl" refers to alkyl substituted with one or more deuterium, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "alkenyl" refers to an alkenyl group, also referred to as an alkene group, wherein the alkene may be further substituted with other related groups.

The term "alkynyl" refers to (CH=C-), wherein the alkynyl may be further substituted by other related groups.
"Hydroxy" refers to -OH.
"Amino" refers to -NH₂.
"Cyano" refers to -CN.
"Nitro" refers to -NO₂.
"Sulfydryl" refers to -SH.
The "carbonyl" refers to -C(O)-.
"Carboxyl" refers to -C(O)OH.
"Oxo" refers to =O.
"NBS" refers to N-bromosuccinimide.
"DCM" refers to dichloromethane.
"Pd(dppf)Cl₂" refers to [1,1'-bis(diphenylphosphino)ferrocene]palladium dichloride.
"NCS" refers to *N*-chlorosuccinimide.
"DMF" refers to *N,N-*dimethylformamide.
"DIEA" refers to *N,N*-diisopropylethylamine.
"MOM" refers to methoxymethyl ether.
"THF" refers to tetrahydrofuran.
"TFA" refers to trifluoroacetic acid.
"AIBN" refers to azobisisobutyronitrile.
"PE" refers to petroleum ether.
"EA" refers to ethyl acetate.

The terms "comprise", "include", "have", "contain", or "relate" and other variations thereof herein are inclusive or open-ended and do not exclude additional unrecited elements or method steps. It should be understood by those skilled in the art that terms described above such as "comprise" encompass the meaning of "consist of'.

The term "one or more" or similar expressions "at least one" may indicate, for example, 1, 2, 3, 4, 5, 6, 7, 8, 9, 10 or more.

When the lower and upper limits of a range of values are disclosed, any value falling within the range and any included range are specifically disclosed. In particular, each range of values disclosed herein is to be understood as indicating each value and range encompassed within the broader range.

As used herein, "Z" and "-Z-" both refer to the same particular group, which may be used interchangeably.

The expression "m-n" as used herein refers to the range of m to n as well as to sub-ranges consisting of point values therein and the point values. For example, the expression "C₂-C₈" or "C₂₋₈" encompasses a range of 2 to 8 carbon atoms and should be understood as also encompassing any sub-range and each point value therein, e.g., C₂-C₅, C₃-C₄, C₂-C₆, C₃-C₆, C₄-C₆, C₅-C₆, C₄-C₇, C₄-C₈, and the like, as well as C₂, C₃, C₄, C₅, C₆, C₇, C₈, and the like. For example, the expression "C₃-C₁₀" or "C₃₋₁₀" should also be understood in a similar manner, for example, it can encompass any sub-range and point value therein, e.g., C₃-C₉, C₆-C₉, C₆-C₈, C₆-C₇, C₇-C₁₀, C₇-C₉, C₇-C₈, C₈-C₉, etc., and C₃, C₄, C₅, C₆, C₇, C₈, C₉, C₁₀, etc. For another example, the expression "C₁-C₆" or "C₁₋₆" encompasses a range of 1 to 6 carbon atoms and should be understood as also encompassing any sub-range and each point value therein, e.g., C₂-C₅, C₃-C₄, C₁-C₂, C₁-C₃, C₁-C₄, C₁-C₅, C₁-C₆, and the like, as well as C₁, C₂, C₃, C₄, C₅, C₆, and the like. For another example, the expression "three to ten membered" should be understood as encompassing any sub-range and each point value therein, e.g., three to five membered, three to six membered, three to seven membered, three to eight membered, four to five membered, four to six membered, four to seven membered, four to eight membered, five to six membered, five to seven membered, five to eight membered, six to seven membered, six to eight membered, nine to ten membered, etc., and three membered, four membered, five membered, six membered, seven membered, eight membered, nine membered, ten membered, etc. Other similar expressions herein should also be understood in a similar manner.

The expressions "X is selected from A, B, or C", "X is selected from A, B, and C", "X is A, B, or C", "X is A, B, and C", and the like as used herein all carry the same meaning, i.e., X may be any one or more of A, B, and C.

The term "optional" or "optionally" refers to that the subsequently described event or circumstance may occur or may not occur. The description includes instances where the event or circumstance occurs and instances where the event or circumstance does not occur. For example, "cycloalkyl optionally substituted with alkyl" means that alkyl may, but does not necessarily exist and that the description includes instances where the cycloalkyl is or is not substituted with alkyl.

The terms "substitution" and "substituted" mean that one or more (e.g., 1, 2, 3, or 4) hydrogens on a specified atom is replaced with a selection from the designated group, with the proviso that the normal valency of the atom specified is not exceeded and that the replacement results in a stable compound. A combination of substituents and/or variables is permissible only if the combination results in a stable compound. When it is stated that a certain substituent is absent, it should be understood that the substituent may be one or more hydrogen atoms, provided that the structure enables the compound to reach a stable state. When it is stated that each carbon atom in a group may optionally be replaced by a heteroatom, the proviso is that the normal valency of all atoms in the group in the present case is not exceeded, and that a stable compound is formed.

If a substituent is described as "optionally substituted with...", the substituent may be unsubstituted or may be substituted. If an atom or group is described as optionally substituted with one or more substituents in the list of substituents, one or more hydrogens on the atom or group can be replaced with an independently selected and optional substituent. When the substituent is oxo (namely =O), it means that two hydrogen atoms are replaced. Unless otherwise indicated, as used herein, the connecting point of a substituent may be from any suitable position of the substituent.

When a bond of a substituent is shown to pass through a bond connecting two atoms in the ring, the substituent may be bonded to any one of the ring-forming atoms in the substitutable ring.

When any variable (e.g., R), as well as labeled variables (e.g., R₁, R₂, R₃, R₄, R₅, R₆, R₇, etc.) occurs more than once in a composition or structure of a compound, the variable is independently defined in each case at each occurrence. For example, if a group is substituted with 0, 1, 2, 3 or 4 R substituents, the group can be optionally substituted with up to four R substituents, and each R substituent is independently defined in each case.

The term "substituted" means that one or more hydrogen atoms on a compound or group are replaced by other atoms or groups, with the proviso that stable valence states or compounds are formed. The expression "unsubstituted" may also be understood as "not substituted". It should be understood that when a substituent is hydrogen, this may also mean that the corresponding group is "unsubstituted" or "not substituted".

The compounds of the present invention may exist in a form of a specific geometric isomer or stereoisomer. All such compounds are contemplated herein, including *cis* and *trans* isomers, (-)- and (+)-enantiomers, (R)- and (S)-enantiomers, diastereoisomers, (D)-isomers, (L)-isomers, and racemic mixtures and other mixtures thereof, such as an enantiomer or diastereomer enriched mixture, all of which are encompassed within the scope of the present invention. Additional asymmetric carbon atoms may be present in substituents such as an alkyl group. All these isomers and mixtures thereof are encompassed within the scope of the present invention. In certain embodiments, preferred compounds are those isomer compounds that exhibit superior biological activity. Purified or partially purified isomers and stereoisomers, or racemic or diastereomeric mixtures of the compound of the present invention are also encompassed within the scope of the present invention. Purification and isolation of such materials can be accomplished by standard techniques known in the art.

All hydrogen atoms described in the present invention may be substituted with isotope deuterium, and any hydrogen atom in the compounds of the examples to which the present invention relates may also be substituted with a deuterium atom.

The term "pharmaceutically acceptable" substance refers to those substances which are, within the scope of sound medical judgment, suitable for use in contact with the tissues of patients without undue toxicity, irritation, allergic response and other problems, commensurate with a reasonable benefit to risk ratio, and effective for their intended use.

The term "pharmaceutically acceptable salt" refers to salts of the compounds of the present invention, which are safe and effective for use in the body of a mammal and possess the requisite biological activities.

The term "pharmaceutical composition" refers to a mixture containing one or more of the compounds described herein or a physiologically/pharmaceutically acceptable salt or pro-drug thereof, and other chemical components, for example physiologically/pharmaceutically acceptable carriers or excipients. The pharmaceutical composition is intended to promote the administration to an organism, so as to facilitate the absorption of the active ingredient, thereby exerting biological activity.

The term "pharmaceutically acceptable carrier" refers to those substances which do not have a significant irritating effect on organisms and do not impair the biological activity and properties of the active compound. The "pharmaceutically acceptable carrier" includes, but is not limited to a glidant, a sweetener, a diluent, a preservative, a dye/colorant, a flavoring agent, a surfactant, a wetting agent, a dispersant, a disintegrant, a stabilizer, a solvent or an emulsifier.

The terms "administration" or "administering" and the like refer to those methods which enable a compound or composition to be delivered to a desired site of biological action. Those methods include, but are not limited to, oral administration or parenteral (including intraventricular, intravenous, subcutaneous, intraperitoneal, intramuscular, intravascular injection or infusion), topical, rectal administrations, and the like. Those methods especially include injection or oral administration.

As used herein, the term "treat", "treating" or "treatment" includes alleviating, reducing, or ameliorating a disease or symptom; preventing other symptoms; ameliorating or preventing metabolic factors underlying a symptom; inhibiting a disease or symptom, e.g., arresting the development of a disease or symptom; alleviating a disease or symptom; promoting the alleviation of a disease or symptom; or halting the signs of a disease or symptom, and extends to include prevention. The "treat", "treating" or "treatment" also includes achieving a therapeutic benefit and/or a prophylactic benefit. The therapeutic benefit refers to eradication or amelioration of a condition being treated. In addition, the therapeutic benefit is achieved by eradicating or ameliorating one or more physiological signs associated with an underlying disease, and amelioration of the underlying disease in the subject is observed, although the subject may still be afflicted with the underlying disease. The prophylactic benefit refers to the use of a composition by a patient to prevent the risk of a disease or the administration of a composition by a patient when the patient develops one or more physiological conditions of a disease, although the disease has not yet been diagnosed.

The term "active ingredient", "therapeutic agent", "active substance" or "active agent" refers to a chemical entity that is effective in treating a target disorder, disease, or condition. The term "nervous and mental disease" refers to a general term for neurological diseases and psychiatric diseases, including neurological diseases and/or psychiatric diseases.

For a drug, drug unit or active ingredient, the term "effective amount", "therapeutically effective amount" or "prophylactically effective amount" refers to an amount of a drug or agent that is sufficient to provide the desired effect with acceptable side effects. The determination of the effective amount varies from person to person. It depends on the age and general condition of a subject, as well as the particular active substance used. The appropriate effective amount in a case may be determined by those skilled in the art in the light of routine tests.

As used herein, an "individual" includes a human or non-human animal. An exemplary human individual includes a human individual (referred to as patients) with a disease (e.g., a disease described herein) or a normal individual. As used herein, a "non-human animal" includes all vertebrates, such as non-mammals (e.g., birds, amphibians, and reptiles) and mammals, such as non-human primates, livestock and/or domesticated animals (e.g., sheep, dogs, cats, cows, pigs, etc.).

The following detailed description is intended to illustrate non-limiting embodiments and to enable others skilled in the art to more fully understand the technical scheme of the present invention, its principles, and its practical application, so that others skilled in the art may modify and implement the present invention in various forms to allow it to be optimally adapted to the particular use contemplated.

### Beneficial Effects

The compounds of the present invention are novel in structure, relatively stable in chemical properties, capable of generating a relatively good anesthetic effect, better in drug effect, and safer, and have a larger safety window and fewer side effects.

### DETAILED DESCRIPTION

The present invention will be further illustrated with reference to the following specific examples. It should be understood that these examples are merely intended to illustrate the present invention rather than limit the scope of the present invention. Furthermore, it should be understood that various changes or modifications of the present invention can be made by those skilled in the art after reading the teachings of the present invention, and these equivalents also fall within the scope of the appended claims of the present application.

### EXAMPLES

Embodiments of the present invention will be described in detail below with reference to examples, but those skilled in the art will appreciate that the following examples are only illustrative of the present invention and should not be construed as limiting the scope of the present invention. Experimental procedures without specified conditions in the examples are conducted according to conventional conditions or conditions recommended by the manufacturer. Reagents or instruments without specified manufacturers used herein are conventional products that are commercially available. The proportions or percentages used herein are calculated by weight, unless otherwise specified.

The compound structure of the present invention is determined by nuclear magnetic resonance (NMR) and/or liquid chromatography-mass spectrometry (LC-MS).

The NMR chemical shift (δ) is given in parts per million (ppm). The NMR determination was conducted by using an AVANCE III 600 nuclear magnetic resonance apparatus or a Bruker Avance III 400 MHz nuclear magnetic resonance apparatus, with deuterated dimethyl sulfoxide (DMSO-*d₆*), deuterated methanol (CD₃OD), or deuterated chloroform (CDCl₃) as determination solvents, and tetramethylsilane (TMS) as an internal standard.

The LC-MS determination was conducted by using a Japan Shimadzu LCMS2020 mass spectrometer. HPLC analysis was performed by using a Japan Shimadzu LC20A liquid chromatograph.

The Yantai Jiangyou silica gel plate was adopted as a thin layer chromatography silica gel plate. The specification adopted by the TLC was 0.2 mm ± 0.03 mm, and the specification adopted by the thin layer chromatography for product separation and purification was 0.4 mm-0.5 mm.

### Example 1 5-isopropyl-3-methyl-2,3-dihydrobenzofuran-4-ol

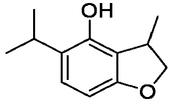

### Synthesis scheme:

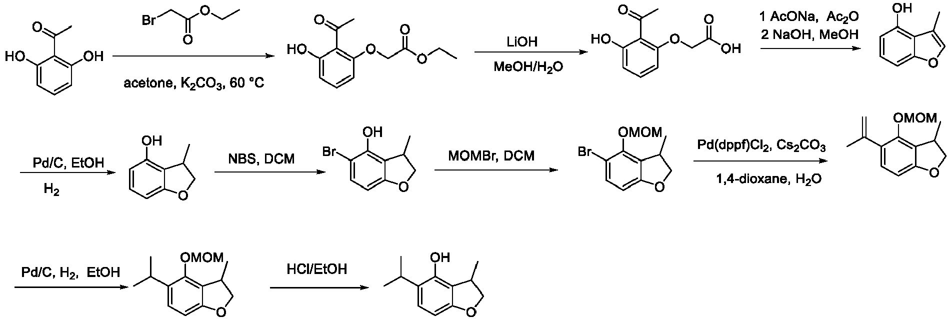

### Step 1: preparation of ethyl 2-(2-acetyl-3-hydroxyphenoxy)acetate

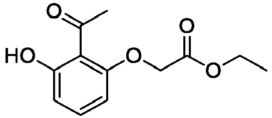

2,6-Dihydroxy acetophenone (15 g, 98.7 mmol), potassium carbonate (33.9 g, 246.0 mmol), and ethyl bromoacetate (11.46 mL, 103.5 mmol) were added to acetone (180 mL), the mixture was heated to 60 °C, reacted for 2 h with magnetic stirring, cooled, and filtered, and the filtrate was concentrated to dryness by rotary evaporation to give the target product (20.85 g, 89% yield).

LC-MS: MS Found: 237 [M-H]⁻.

### Step 2: preparation of 2-(2-acetyl-3-hydroxyphenoxy)acetic acid

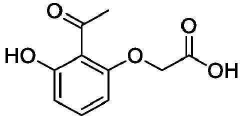

Ethyl 2-(2-acetyl-3-hydroxyphenoxy)acetate (20.0 g, 95.1 mmol) and lithium hydroxide (12.0 g, 285.3 mmol) were added to a mixed solvent of MeOH/H₂O (400 mL/100 mL), the mixture was stirred for reaction at room temperature for 2 h. Methanol was removed under reduced pressure, the remaining solution was adjusted to pH 3 with a 1 M HCl solution, water (100 mL) was added, and the mixture was extracted with ethyl acetate (300 mL × 2). The organic phases were combined and washed with water (200 mL ×2), washed with saturated brine (200 mL × 2), dried over anhydrous sodium sulfate, and filtered, and the filtrate was concentrated under reduced pressure to give the target product (14 g, 79% yield).

LC-MS: MS Found: 209 [M-H]⁻.

### Step 3: preparation of 3-methylbenzofuran-4-ol

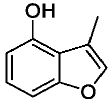

2-(2-Acetyl-3-hydroxyphenoxy)acetic acid (14.0 g, 66.6 mmol) and sodium acetate (38.0 g, 466 mmol) were added to acetic anhydride (110 mL), the mixture was heated, refluxed, and reacted for 2 h. The mixture was cooled to room temperature, added to ice water, filtered, and dissolved in methanol (100 mL). A sodium hydroxide solution (2 M, 100 mL) was added, and the mixture was heated, refluxed, and reacted for 30 min. After the mixture returned to room temperature, the mixture was poured into water (500 mL), adjusted to pH 3.0 with a 2 M hydrochloric acid solution, and filtered to give the target product (8 g, 80% yield).

LC-MS: MS Found: 147 [M-H]⁻.

### Step 4: preparation of 3-methyl-2,3-dihydrobenzofuran-4-ol

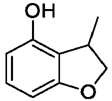

3-Methylbenzofuran-4-ol (8 g, 53.3 mmol) and Pd/C (800 mg) were added to ethanol (200 mL). The mixture was reacted at room temperature under hydrogen atmosphere (1 atm) overnight and filtered, and the filtrate was concentrated under reduced pressure to give a crude product, which was purified by normal-phase silica gel column chromatography (petroleum ether:ethyl acetate = 10:1) to give the target product (6 g, 80% yield).

LC-MS: MS Found: 149 [M-H]⁻.

### Step 5: preparation of 5-bromo-3-methyl-2,3-dihydrobenzofuran-4-ol

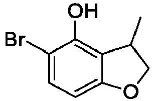

3-Methyl-2,3-dihydrobenzofuran-4-ol and NBS (2.1 g, 11.9 mmol) were sequentially added to ethyl acetate (40 mL) at 0 °C. The mixture was warmed to room temperature and reacted for 16 h and filtered, and the filtrate was concentrated to give a crude product, which was purified by reversed-phase column chromatography to give the target product (1.0 g, 36% yield).

LC-MS: MS Found: 227 [M-H]⁻.

### Step 6: preparation of 5-bromo-4-(methoxymethoxy)-3-methyl-2,3-dihydrobenzofuran

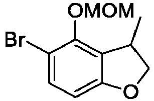

5-Bromo-3-methyl-2,3-dihydrobenzofuran-4-ol (1.0 g, 4.3 mmol) was dissolved in dichloromethane (20 mL). Bromomethyl methyl ether (545 mg, 4.3 mmol) and diisopropylethylamine (1.1 g, 8.6 mmol) were added, and the mixture was reacted at room temperature for 2 h. Water (20 mL) was added, and the mixture was extracted with ethyl acetate (10 mL × 3). The organic phases were combined and then washed with water (10 mL × 3), washed with saturated brine (10 mL × 3), dried over anhydrous Na₂SO₄, filtered, and concentrated under reduced pressure to give a crude product, which was purified by normal-phase silica gel column chromatography (petroleum ether:ethyl acetate = 30:1) to give the target product (1.0 g, 83% yield).

LC-MS: MS Found: 273 [M+H]⁺.

### Step 7: preparation of 4-(methoxymethoxy)-3-methyl-5-(propyl-1-en-2-yl)-2,3-dihydrobenzofuran

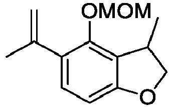

5-Bromo-4-(methoxymethoxy)-3-methyl-2,3-dihydrobenzofuran (1.0 g, 3.66 mmol) and isopropenylboronic acid pinacol ester (1.66 g, 9.78 mmol) were dissolved in 1,4-dioxane (20 mL). Water (2 mL), Pd(dppf)Cl₂ (100 mg, 0.2 mmol), and cesium carbonate (3.58 g, 10.9 mmol) were added, and the mixture was heated to 100 °C and reacted for 16 h under nitrogen atmosphere. After the reaction was completed, the mixture was cooled to room temperature, added with water (20 mL), and extracted with ethyl acetate (20 mL × 3), and the organic phases were combined and concentrated to give a crude product, which was purified by normal-phase silica gel column chromatography to give the target product (800 mg, 93% yield).

LC-MS: MS Found: 235 [M+H]⁺.

### Step 8: preparation of 5-isopropyl-4-(methoxymethoxy)-3-methyl-2,3-dihydrobenzofuran

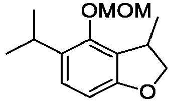

4-(Methoxymethoxy)-3-methyl-5-(propyl-1-en-2-yl)-2,3-dihydrobenzofuran (200 mg, 0.854 mmol) and Pd/C (40 mg) were sequentially added to ethanol (10 mL) at room temperature The mixture was reacted under H₂ atmosphere (1 atm) for 1 h and filtered, and the filtrate was concentrated to give the target product (150 mg, 75% yield).

LC-MS: MS Found: 237 [M+H]⁺.

### Step 9: preparation of 5-isopropyl-3-methyl-2,3-dihydrobenzofuran-4-ol

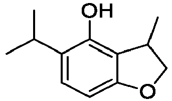

5-Isopropyl-4-(methoxymethoxy)-3-methyl-2,3-dihydrobenzofuran (150 mg, 0.653 mmol) was added to a HCl/EtOH (4 M, 3 mL) solution, and the mixture was reacted at room temperature for 1 h and concentrated to give a crude product, which was purified by reversed-phase preparative HPLC to give the target final product, **Example 1** (53.2 mg, 44% yield).

LC-MS: MS Found: 191 [M-H]⁻.

¹H NMR (400 MHz, CDCl₃): δ 6.43 (d, J = 8.0 Hz, 1H), 6.38 (d, J = 8.0 Hz, 1H), 4.62 (dd, J = 8.8 5.2 Hz, 1H), 4.15 (dd, J = 8.8, 5.2 Hz, 1H), 3.60-3.55 (m, 1H), 3.03-3.00 (m, 1H), 1.36 (d, J = 6.4 Hz, 3H), 1.24 (dd, J = 8.8, 7.2 Hz, 6H).

### Example 2 5-isopropyl-3-methyl-2,3-dihydro-1H-inden-4-ol

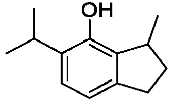

### Synthesis scheme:

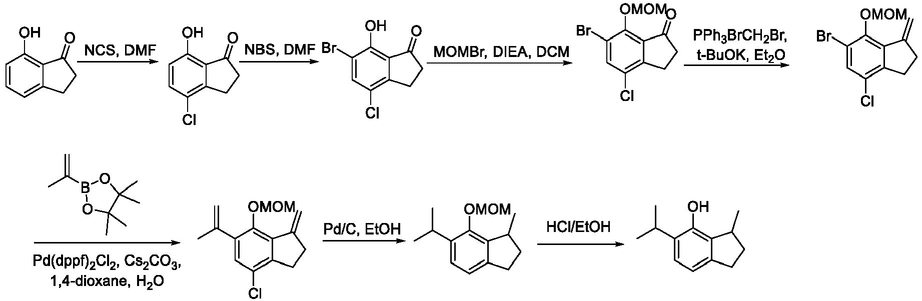

### Step 1: preparation of 4-chloro-7-hydroxy-2,3-dihydro-1H-inden-1-one

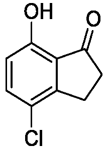

N-Chlorosuccinimide (3.6 g, 27.0 mmol) was added to a solution of 7-hydroxy-2,3-dihydro-1H-inden-1-one (4.0 g, 27.0 mmol) in *N,N*-dimethylformamide (80 mL) at 0 °C. The reaction mixture was stirred at room temperature for 16 h and filtered, and the filtrate was concentrated under reduced pressure to give a crude product, which was purified by a reversed-phase column to give the target product (2.0 g, 40% yield).

LC-MS: MS Found: 181 [M-H]⁻.

### Step 2: preparation of 6-bromo-4-chloro-7-hydroxy-2,3-dihydro-1H-inden-1-one

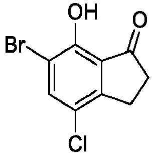

4-Chloro-7-hydroxy-2,3-dihydro-1*H*-inden-1-one (2.0 g, 10.9 mmol) and N-bromosuccinimide (1.9 g, 10.9 mmol) were dissolved in *N,N*-dimethylformamide (20 mL). The mixture was stirred at room temperature for 2 h, the reaction mixture was filtered, and the filtrate was concentrated under reduced pressure to give a crude product, which was purified by a reversed-phase column to give the target product (1.8 g, 64% yield).

LC-MS: MS Found: 259 [M-H]⁻.

### Step 3: preparation of 6-bromo-4-chloro-7-(methoxymethoxy)-2,3-dihydro-1H-inden-1-one

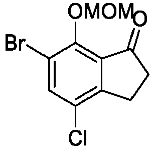

Bromomethyl methyl ether (1.0 g, 7.6 mmol) and *N*,*N*-diisopropylethylamine (2.6 g, 20.6 mmol) were added to a solution of 6-bromo-4-chloro-7-hydroxy-2,3-dihydro-1*H*-inden-1-one (1.8 g, 6.9 mmol) in dichloromethane (10 mL), and the reaction mixture was stirred at room temperature for 16 h, added with water (20 mL), and extracted with ethyl acetate. The organic phases were combined, washed with water (10 mL × 2) and brine (10 mL × 2), dried over anhydrous sodium sulfate, and filtered, and the filtrate was concentrated under reduced pressure to give a crude product, which was purified by a normal-phase silica gel column (petroleum ether:ethyl acetate = 10:1) to give the target product (1.7 g, 55% yield).

LC-MS: MS Found: 305 [M+H]⁺.

### Step 4: preparation of 6-bromo-4-chloro-7-(methoxymethoxy)-1-methylene-2,3-dihydro-1H-indene

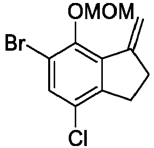

After a solution of methyl triphenyl phosphonium bromide (2.3 g, 6.9 mmol) and potassium *tert-*butoxide (749 mg, 6.9 mmol) in diethyl ether (20 mL) was stirred at room temperature for 15 min, 6-bromo-4-chloro-7-(methoxymethoxy)-2,3-dihydro-1*H*-inden-1-one (1.7 g, 5.6 mmol) was added, and the mixture was stirred at room temperature for 16 h. The reaction mixture was concentrated under reduced pressure to give a crude product, which was purified by a normal-phase silica gel column (petroleum ether:ethyl acetate = 50:1) to give the target product (1.1 g, 65% yield).

LC-MS: MS Found: 303 [M+H]⁺.

### Step 5: preparation of 4-chloro-7-(methoxymethoxy)-1-methylene-6-(prop-1-en-2-yl)-2,3-dihydro-1H-indene

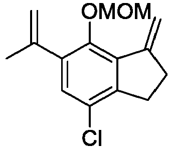

[1,1'-bis(diphenylphosphino)ferrocene]palladium dichloride (60 mg, 0.2 mmol) and cesium carbonate (1.9 g, 5.93 mmol) were added to a mixed solution of 6-bromo-4-chloro-7-(methoxymethoxy)-1-methylene-2,3-dihydro-1*H*-indene (600 mg, 1.98 mmol) and isopropenylboronic acid pinacol ester (890 mg, 5.28 mmol) in 1,4-dioxane (10 mL) and water (2 mL). The reaction mixture was stirred at 100 °C for 16 h under nitrogen atmosphere. After the reaction mixture was cooled to room temperature, water (10 mL) was added and the mixture was extracted with ethyl acetate (10 mL × 3). The combined organic phases were dried over anhydrous sodium sulfate, filtered, and concentrated under reduced pressure to give a crude product, which was purified by a silica gel column (petroleum ether:ethyl acetate = 50:1) to give the target product (400 mg, 76% yield).

LC-MS: MS Found: 265 [M+H]⁺.

### Step 6: 6-isopropyl-7-(methoxymethoxy)-1-methyl-2,3-dihydro-1H-indene

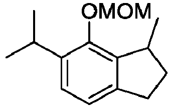

A solution of 4-chloro-7-(methoxymethoxy)-1-methylene-6-(prop-1-en-2-yl)-2,3-dihydro-1*H-*indene (400 mg, 1.51 mmol) and palladium on carbon (40 mg) in ethanol (8 mL) was stirred at room temperature under hydrogen atmosphere (1 atm) overnight. The reaction mixture was filtered through celite, and the filtrate was concentrated *in vacuo* to give the target product (280 mg, 83% yield).

LC-MS: MS Found: 235 [M+H]⁺.

### Step 7: 5-isopropyl-3-methyl-2,3-dihydro-1H-inden-4-ol

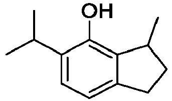

Concentrated hydrochloric acid (12 M, 0.2 mL) was added to a solution of 6-isopropyl-7-(methoxymethoxy)-1-methyl-2,3-dihydro-1*H*-indene (200 mg, 0.13 mmol) in ethanol (5 mL) at room temperature. The reaction mixture was stirred at room temperature for 15 min. The reaction mixture was concentrated *in vacuo* to give a crude product, which was purified by reversed-phase preparative HPLC to give the target final product, **Example 2** (66.7 mg, 41% yield).

LC-MS: MS Found: 189 [M-H]⁻.

¹H NMR (400 MHz, CDCl₃): δ 7.01 (d, J = 7.6 Hz, 1H), 6.78 (d, J = 7.6 Hz, 1H), 4.56 (s, 1H), 3.35-3.31 (m, 1H), 3.20-3.13 (m, 1H), 2.98-2.94 (m, 1H), 2.81-2.74 (m, 1H), 2.33-2.24 (m, 1H), 1.78-1.71 (m, 1H), 1.28-1.25 (m, 9H).

### Example 3 5-(1-cyclopropylethyl)-3-methyl-2,3-dihydrobenzofuran-4-ol

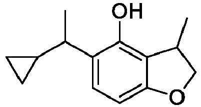

### Synthesis scheme

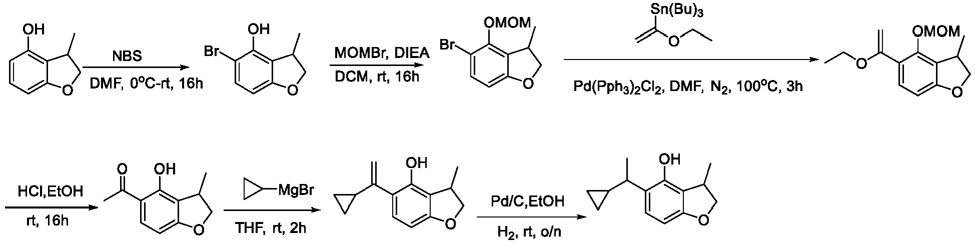

### Step 1: preparation of 5-bromo-3-methyl-2,3-dihydrobenzofuran-4-ol

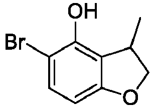

N-Bromosuccinimide (5.89 g, 27.0 mmol) was added to a solution of 3-methyl-2,3-dihydrobenzofuran-4-ol (5.0 g, 33.3 mmol) in *N,N*-dimethylformamide (80 mL) at 0 °C. The reaction mixture was stirred at room temperature for 16 h. The reaction mixture was filtered, and the filtrate was concentrated under reduced pressure to give a crude product, which was purified by a reversed-phase column to give the target product (5.0 g, 65% yield).

LC-MS: MS Found: 227 [M-H]⁻.

### Step 2: preparation of 5-bromo-4-(methoxymethoxy)-3-methyl-2,3-dihydrobenzofuran

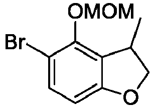

Bromomethyl methyl ether (2.8 g, 22.8 mmol) and *N,N*-diisopropylethylamine (8.4 g, 65.4 mmol) were added to a solution of 5-bromo-3-methyl-2,3-dihydrobenzofuran-4-ol (5.0 g, 21.8 mmol) in dichloromethane (100 mL). The reaction mixture was stirred at room temperature for 16 h. Water (20 mL) was added to the reaction mixture and the mixture was extracted with ethyl acetate (10 mL × 2). The combined organic phases were washed with water (10 mL × 2) and brine (10 mL × 2), dried over anhydrous sodium sulfate, filtered, and concentrated under reduced pressure to give a crude product, which was purified by a silica gel column (petroleum ether:ethyl acetate = 10:1) to give the target product (5.2 g, 87% yield).

LC-MS: MS Found: 273 [M+H]⁺.

### Step 3: preparation of 5-(1-ethoxyvinyl)-4-(methoxymethoxy)-3-methyl-2,3-dihydrobenzofuran

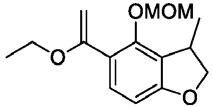

bis(Triphenylphosphine)palladium(II) dichloride (511 mg, 0.73 mmol) was added to a solution of 5-bromo-4-(methoxymethoxy)-3-methyl-2,3-dihydrobenzofuran (4.0 g, 14.6 mmol) and tributyl(1-ethoxyvinyl)tin (5.2 g, 14.6 mmol) in *N*,*N*-dimethylformamide. The reaction mixture was stirred at 100 °C for 3 h under nitrogen atmosphere. After the reaction mixture was cooled to room temperature, water (20 mL) was added and the mixture was extracted with ethyl acetate EA (10 mL × 2). The combined organic phases were washed with water (10 mL × 2) and brine (10 mL × 2), dried over anhydrous sodium sulfate, filtered, and concentrated under reduced pressure to give a crude product, which was purified by a normal-phase silica gel column (petroleum ether:ethyl acetate = 10:1) to give the target product (3.4 g, 88% yield).

LC-MS: MS Found: 265 [M+H]⁺.

### Step 4: preparation of 1-(4-hydroxy-3-methyl-2,3-dihydrobenzofuran-5-yl)ethanone

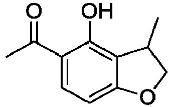

Concentrated hydrochloric acid (10 mL) was added to a solution of 5-(1-ethoxyvinyl)-4-(methoxymethoxy)-3-methyl-2,3-dihydrobenzofuran (3.4 g, 12.87 mmol) in ethanol (60 mL). The reaction mixture was stirred at room temperature for 16 h. Water (20 mL) was added to the reaction mixture and the mixture was extracted with ethyl acetate EA (10 mL × 2). The combined organic phases were washed with water (10 mL × 2) and brine (10 mL × 2), dried over anhydrous sodium sulfate, filtered, and concentrated under reduced pressure to give a crude product, which was purified by a silica gel column (petroleum ether:ethyl acetate = 15:1) to give the target product (1.8 g, 73% yield).

LC-MS: MS Found: 193 [M+H]⁺.

### Step 5: preparation of 5-(1-cyclopropylvinyl)-3-methyl-2,3-dihydrobenzofuran-4-ol

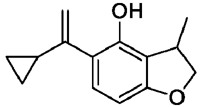

A solution of 1-(4-hydroxy-3-methyl-2,3-dihydrobenzofuran-5-yl)ethanone (1 g, 5.2 mmol) in tetrahydrofuran was stirred at 0 °C for 5 min. Then a solution of cyclopropylmagnesium bromide in THF (1 M, 20.8 mL, 20.8 mmol) was added, and the mixture was stirred at room temperature for 2 h. Water (10 mL) was added to the reaction mixture and the mixture was extracted with ethyl acetate (5 mL × 2). The combined organic phases were washed with water (5 mL × 2) and brine (5 mL × 2), dried over anhydrous sodium sulfate, filtered, concentrated under reduced pressure, and purified by a silica gel column (petroleum ether:ethyl acetate = 20:1) to give the target product (700 mg, 62% yield).

LC-MS: MS Found: 217 [M+H]⁺.

### Step 6: preparation of 5-(1-cyclopropylethyl)-3-methyl-2,3-dihydrobenzofuran-4-ol

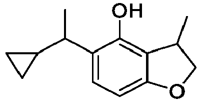

A solution of 5-(1-cyclopropylvinyl)-3-methyl-2,3-dihydrobenzofuran-4-ol (400 mg, 1.85 mmol) and palladium on carbon (100 mg) in ethanol (8 mL) was stirred at room temperature under hydrogen atmosphere (1 atm) overnight. The reaction mixture was filtered through celite, and the filtrate was concentrated *in vacuo* and purified by reversed-phase preparative HPLC to give the target final product, **Example 3** (29.8 mg, 7% yield).

LC-MS: MS Found: 217 [M-H]⁻.

¹H NMR (400 MHz, CDCl₃): δ 6.98 (d, J = 8.4 Hz, 1H), 6.38 (d, J = 8.4 Hz, 1H), 4.86 (d, J = 5.6 Hz, 1H), 4.65-4.60 (m, 1H), 4.16-4.13 (m, 1H), 3.62-3.54 (m, 1H), 2.44-2.36 (m, 1H), 1.36 (dd, J = 6.8, 2.0 Hz, 3H), 1.28-1.25 (m, 3H), 1.06-0.96 (m, 1H), 0.59-0.52 (m, 1H), 0.50-0.43(m, 1H), 0.24-0.12 (m, 2H).

### Example 4 5-(1-cyclopropylethyl)-7-fluoro-3-methyl-2,3-dihydrobenzofuran-4-ol

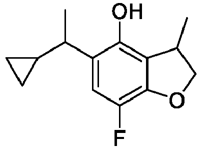

### Synthesis scheme:

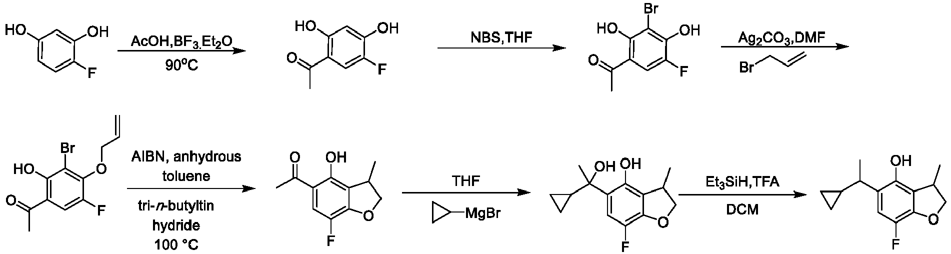

### Step 1: preparation of 1-(5-fluoro-2,4-dihydroxyphenyl)ethan-1-one

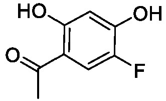

4-Fluorobenzene-1,3-diol (14 g, 109 mmol) was dissolved in a solution of boron trifluoride diethyl etherate (84 mL). Acetic acid (12 mL) was added dropwise, and the mixture was reacted at 90 °C for 5 h. After the reaction was completed, the mixture was poured into a 10% aqueous sodium acetate solution (300 mL) and extracted with ethyl acetate (500 mL). The organic phase was washed with an aqueous sodium bicarbonate solution (200 mL), dried, concentrated, and then slurried with petroleum ether/dichloromethane (10/1, 50 mL) to give the target product (16 g, 86% yield).

LC-MS: MS Found: 169 [M-H]⁻.

¹H NMR (600 MHz, DMSO): δ 12.31 (s, 1H), 11.22 (s, 1H), 7.70 (d, J = 12.0 Hz, 1H), 6.42 (d, J = 6.0 Hz, 1H), 2.52 (s, 3H).

### Step 2: preparation of 1-(3-bromo-5-fluoro-2,4-dihydroxyphenyl)ethan-1-one

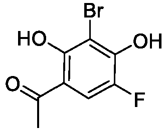

1-(5-Fluoro-2,4-dihydroxyphenyl)ethan-1-one (16 g, 94 mmol) was dissolved in tetrahydrofuran (320 mL). NBS (16.75 g, 94 mmol) was added at room temperature and the mixture was stirred for 3 h, after which the reaction was quenched with water. The mixture was extracted, dried, and concentrated to give the target product (32 g).

LC-MS: MS Found: 247 [M-H]⁻.

¹H NMR (600 MHz, DMSO): δ 13.25 (s, 1H), 11.88 (s, 1H), 7.88 (d, J = 12.0 Hz, 1H), 2.59 (s, 3H).

### Step 3: preparation of 1-(4-(allyloxy)-3-bromo-5-fluoro-2-hydroxyphenyl)ethan-1-one

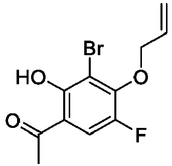

1-(3-Bromo-5-fluoro-2,4-dihydroxyphenyl)ethan-1-one (32 g, 129 mmol) and 3-bromo-1-ene (15.48 g, 129 mmol) were dissolved in DMF (400 mL). Silver carbonate (35.58 g, 129 mmol) was added, and the mixture was stirred at room temperature for two days. The mixture was filtered through celite to give a clear solution, which was diluted with ethyl acetate and extracted with saturated sodium chloride. The organic phase was dried over anhydrous sodium sulfate, concentrated, and purified by a silica gel column (petroleum ether/ethyl acetate = 3%) to give the target product (13 g, 22% yield).

LC-MS: MS Found: 287 [M-H]⁻.

¹H NMR (600 MHz, CDCl₃): δ 12.97 (s, 1H), 7.46 (d, J = 12.0 Hz, 1H), 6.11- 6.04 (m, 1H), 5.45 (d, J = 18.0 Hz, 1H), 5.30 (d, J = 12.0 Hz, 1H), 4.81 (d, J = 6.0 Hz, 2H), 2.59 (s, 3H).

### Step 4: preparation of 1-(7-fluoro-4-hydroxy-3-methyl-2,3-dihydrobenzofuran-5-yl)ethan-1-one

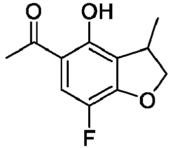

1-(4-(Allyloxy)-3-bromo-5-fluoro-2-hydroxyphenyl)ethan-1-one (13 g, 47 mmol) was dissolved in anhydrous toluene (100 mL). AIBN (8.89 g, 54 mmol) and tri-n-butyltin hydride (26 g, 89 mmol) were added sequentially, and the mixture was heated at 100 °C for 2 h under nitrogen atmosphere. After the reaction was completed, the mixture was diluted with ethyl acetate (300 mL) and extracted with saturated sodium chloride. The organic phase was dried over anhydrous sodium sulfate, concentrated, and purified by a silica gel column (PE/EA = 4%) to give the target product (8.6 g, 91% yield).

LC-MS: MS Found: 209 [M-H]⁻.

¹H NMR (600 MHz, CDCl₃): δ 12.51 (s, 1H), 7.32 (d, J = 12.0 Hz, 1H), 4.85 (t, J = 9.0 Hz, 1H), 4.36 (dd, J =5.4, 8.4 Hz, 1H), 3.75 - 3.69 (m, 1H), 2.53 (s, 3H), 1.40 (d, J = 6.0 Hz, 3H).

### Step 5: preparation of 5-(1-cyclopropyl-1-hydroxyethyl)-7-fluoro-3-methyl-2,3-dihydrobenzofuran-4-ol

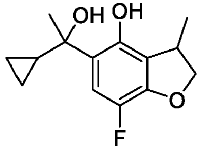

1-(7-Fluoro-4-hydroxy-3-methyl-2,3-dihydrobenzofuran-5-yl)ethan-1-one (8.6 g, 41 mmol) was dissolved in tetrahydrofuran (28 mL). 1 M cyclopropylmagnesium bromide (143 mL) was added under an ice bath, and the mixture was stirred at room temperature for 1 h, after which the reaction was quenched with an aqueous ammonium chloride solution. The mixture was extracted with ethyl acetate (200 mL), and the organic phase was dried over anhydrous sodium sulfate, concentrated, and purified by a silica gel column (petroleum ether/ethyl acetate = 12%) to give the target product (8 g, 78% yield).

LC-MS: MS Found: 251 [M-H]⁻.

### Step 6: preparation of 5-(1-cyclopropylethyl)-7-fluoro-3-methyl-2,3-dihydrobenzofuran-4-ol

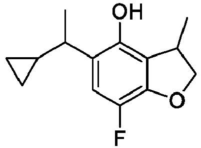

5-(1-Cyclopropyl-1-hydroxyethyl)-7-fluoro-3-methyl-2,3-dihydrobenzofuran-4-ol (8 g, 34 mmol) was dissolved in dichloromethane (400 mL). Triethylsilane (14.79 g, 127 mmol) was added dropwise under an ice bath. After the mixture was stirred for 10 min, trifluoroacetic acid (28.79 g, 253 mmol) was added dropwise, and the mixture was stirred for another 1 h, after which the reaction was quenched with water. The mixture was extracted, and the organic phase was collected. Tetrabutylammonium fluoride (8.9 g, 34 mmol) was added, and the mixture was stirred at room temperature for half an hour. The reaction solution was washed with saturated brine, dried over anhydrous sodium sulfate, concentrated, and purified by a silica gel column (petroleum ether/ethyl acetate = 3%) to give the target final product, **Example 4** (4 g, 53% yield). LC-MS: MS Found: 235 [M-H]⁻.

¹H NMR (600 MHz, CDCl₃, diastereoisomer 1:1): δ 6.82 (d, J = 6.0 Hz, 1H), 6.81 (d, J = 6.0 Hz, 1H), 4.73 - 4.69 (m, 2H), 4.65 (s, 1H), 4.63 (s, 1H), 4.26 - 4.24 (m, 2H), 3.65 - 3.58 (m, 2H), 2.40 - 2.34 (m, 2H), 1.38 - 1.36 (m, 6H), 1.27 - 1.24 (m, 6H), 1.02 - 0.93 (m, 2H), 0.61 - 0.52 (m, 2H), 0.51 - 0.42 (m, 2H), 0.23 - 0.19 (m, 2H), 0.18 - 0.10 (m, 2H).

### Example 5 7-chloro-5-isopropyl-3-methyl-2,3-dihydrobenzofuran-4-ol

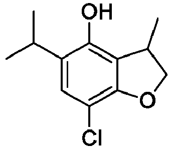

### Synthesis scheme:

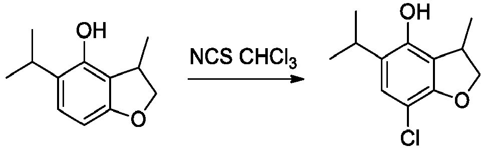

Example 1 was used as the starting material. 5-Isopropyl-3-methyl-2,3-dihydrobenzofuran-4-ol (400 mg, 2.08 mmol) was dissolved in CHCl₃ (10 mL), NCS (556 mg, 4.16 mmol) was added, and the mixture was refluxed and reacted for 2 h. After the reaction was completed, the mixture was cooled and concentrated to give a crude product, which was purified by preparative HPLC to give the target product, **Example 5** (19.5 mg, 4% yield).

LC-MS: MS Found: 225 [M-H]⁻.

¹H NMR (400 MHz, CDCl₃): δ 6.92 (s, 1H), 6.38 (d, J = 8.0 Hz, 1H), 4.71 (dd, J = 8.8, 5.2 Hz, 1H), 4.23 (dd, J = 8.8, 5.2 Hz, 1H), 3.66-3.61 (m, 1H), 3.01-2.95 (m, 1H), 1.36 (d, J = 7.2 Hz, 3H), 1.24 (dd, J = 8.8, 7.2 Hz, 6H).

### Example 6 7-fluoro-5-isopropyl-3,3-dimethyl-2,3-dihydrobenzofuran-4-ol

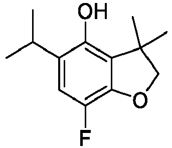

For the synthesis method of Example 6, reference was made to Example 4.

LC-MS: MS Found: 223 [M-H]⁻.

¹H NMR (600 MHz, CDCl₃): δ 6.73 (d, *J* = 11.4 Hz, 1H), 4.48 (s, 1H), 4.28 (s, 2H), 2.98 - 2.91 (m, 1H), 1.46 (s, 6H), 1.23 (d, *J* = 6.6 Hz,6H).

### Example 7 7-fluoro-5-isopropyl-3-methyl-2,3-dihydrobenzofuran-4-ol

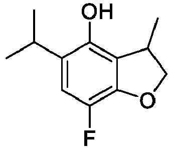

For the synthesis method of Example 7, reference was made to Example 4.

LC-MS: MS Found: 209 [M-H]⁻.

¹H NMR (400 MHz, CDCl₃): δ 6.74 (d, J = 12.0 Hz, 1H), 4.70 (dd, J = 8.8, 5.2 Hz, 1H), 4.24 (dd, J = 8.8, 5.2 Hz, 1H), 3.64-3.58 (m, 1H), 3.04-2.97 (m, 1H), 1.36 (d, J = 6.8 Hz, 3H), 1.24 (dd, J = 8.0, 6.8 Hz, 6H).

### Example 8 5-(1-cyclopropylethyl)-7-fluoro-3,3-dimethyl-2,3-dihydrobenzofuran-4-ol

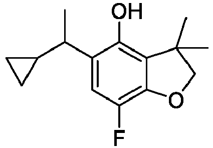

For the synthesis method of Example 8, reference was made to Example 4. LC-MS: MS Found: 249 [M-H]⁻.

¹H NMR (600 MHz, CDCl₃): δ 6.79 (d, *J =* 11.4 Hz, 1H), 4.70 (s, 1H), 4.28 (s, 2H), 2.41 - 2.36 (m, 1H), 1.46 (d, *J* = 3.0 Hz, 6H), 1.25 (d, *J* = 6.6 Hz,3H),1.00 - 0.95 (m, 1H), 0.60 - 0.55 (m, 1H), 0.52 - 0.47 (m,1H), 0.22 - 0.18 (m, 1H), 0.16 - 0.12 (m, 1H).

### Example 9 5-(1-cyclopropylethyl)-3-ethyl-7-fluoro-2,3-dihydrobenzofuran-4-ol

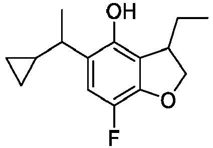

For the synthesis method of Example 9, reference was made to Example 4.

LC-MS: MS Found: 249 [M-H]⁻.

¹H NMR (600 MHz, CDCl₃, diastereoisomer 1:1): δ 6.83 (s, 1H), 6.81 (s, 1H), 4.68 - 4.62 (m, 4H), 4.42 - 4.40 (m, 2H), 3.51 - 3.46 (m, 2H), 2.41 - 2.31 (m, 2H), 1.27 - 1.24 (m, 10H), 0.96 - 0.93 (m, 6H), 0.89 - 0.84 (m, 2H), 0.59 - 0.54 (m, 2H), 0.49 - 0.45 (m, 2H), 0.23 - 0.18 (m, 2H), 0.16 - 0.13 (m, 2H).

### Example 10 Four optical isomers of sodium (5-(1-cyclopropyl)-7-fluoro-3-methyl-2,3-dihydrobenzofuran-4-yl)oxymethyl phosphate (10A1, 10A2, 10B1, and 10B2)

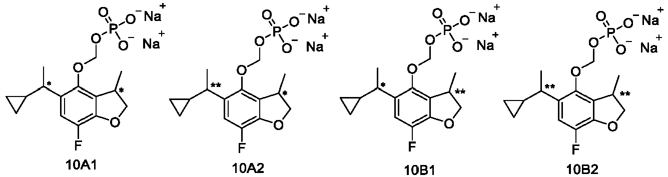

### Synthesis scheme A:

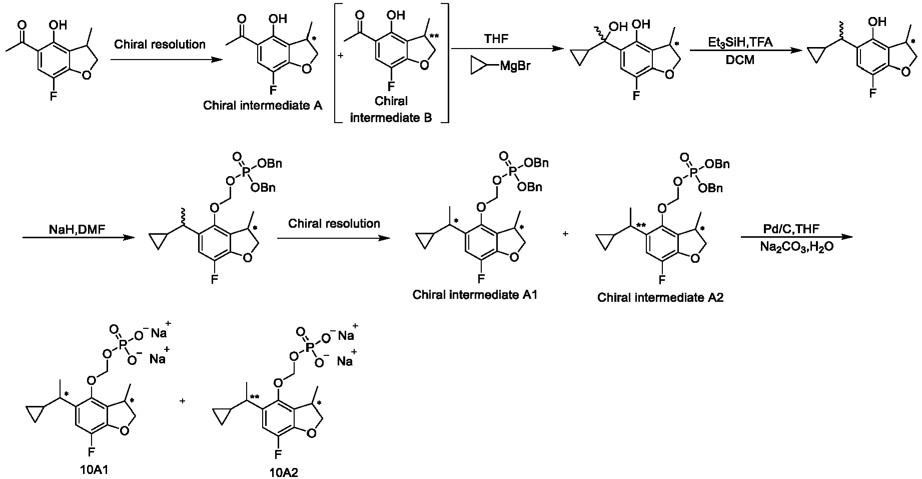

### Step 1: chiral resolution of 1-(7-fluoro-4-hydroxy-3-methyl-2,3-dihydrobenzofuran-5-yl)ethan-1-one

80 g of 1-(7-fluoro-4-hydroxy-3-methyl-2,3-dihydrobenzofuran-5-yl)ethan-1-one was resolved by chiral HPLC to give **chiral intermediate** A (30.5 g, retention time 5.898 min) and **chiral intermediate B** (29.5 g, retention time 6.858 min). Resolution conditions: instrument: Shimadzu LC-20AP; column: DAICEL CHIRALPAK^{®}IA (250 × 25 mm 10 µm); mobile phase: n-hexane/ethanol (0.1% 7 M NH₃ in MeOH) = 80/20; wavelength: 214 nm; flow rate: 30 mL/min; temperature: RT; time: 4.5 min.

### Step 2: synthesis of 5-(1-cyclopropyl-1-hydroxyethyl)-7-fluoro-3-methyl-2,3-dihydrobenzofuran-4-ol

1-(7-Fluoro-4-hydroxy-3-methyl-2,3-dihydrobenzofuran-5-yl)ethan-1-one (29 g, 138 mmol, **chiral intermediate A**) was dissolved in tetrahydrofuran (94 mL). 1 M cyclopropylmagnesium bromide (482 mL) was added under an ice bath, and the mixture was stirred at room temperature for 1 h, after which the reaction was quenched with an aqueous ammonium chloride solution. The mixture was extracted with ethyl acetate (800 mL), and the organic phase was dried over anhydrous sodium sulfate, concentrated, and purified by a silica gel column (PE/EA = 12%) to give the target product of 5-(1-cyclopropyl-1-hydroxyethyl)-7-fluoro-3-methyl-2,3-dihydrobenzofuran-4-ol (26 g, 75% yield).

LC-MS(m/z): [M-H] ⁻ 251.00.

### Step 3: synthesis of 5-(1-cyclopropylethyl)-7-fluoro-3-methyl-2,3-dihydrobenzofuran-4-ol

5-(1-Cyclopropyl-1-hydroxyethyl)-7-fluoro-3-methyl-2,3-dihydrobenzofuran-4-ol (26 g, 110.5 mmol) was dissolved in dichloromethane (1300 mL). Triethylsilane (48.07 g, 413 mmol) was added dropwise under an ice bath. After the mixture was stirred for 10 min, trifluoroacetic acid (93.57 g, 822 mmol) was added dropwise, and the mixture was stirred for another 1 h, after which the reaction was quenched with water. The mixture was extracted, and the organic phase was collected. Tetrabutylammonium fluoride (28.9 g, 110.5 mmol) was added, and the mixture was stirred at room temperature for half an hour. The reaction solution was washed with saturated brine, dried over anhydrous sodium sulfate, concentrated, and purified by a silica gel column (PE/EA = 3%) to give the target crude product of 5-(1-cyclopropylethyl)-7-fluoro-3-methyl-2,3-dihydrobenzofuran-4-ol (27 g).

LC-MS(m/z): [M-H] ⁻ 235.00.

### Step 4: synthesis of methyl dibenzyl[(5-(1-cyclopropylethyl)-7-fluoro-3-methyl-2,3-dihydro-1-benzofuran-4-yl)oxy]phosphate

5-(1-Cyclopropylethyl)-7-fluoro-3-methyl-2,3-dihydrobenzofuran-4-ol (27 g) was dissolved in anhydrous DMF (400 mL). Sodium hydride (5.4 g, 135 mmol) was added under an ice bath. The mixture was stirred for another half an hour under an ice bath. Dibenzylchloromethyl phosphate (30 g, 92 mmol) was added, and the mixture was warmed to room temperature and then stirred overnight. After the reaction was completed, the reaction was quenched with a saturated ammonium chloride solution. The mixture was extracted with ethyl acetate (600 mL), and the organic phase was dried over anhydrous sodium sulfate, concentrated, and purified by a silica gel column (PE/EA = 20%) to give the target product of methyl dibenzyl[(5-(1-cyclopropylethyl)-7-fluoro-3-methyl-2,3-dihydro- 1-benzofuran-4-yl)oxy]phosphate.

LC-MS(m/z): [M+H] ⁺ 527.10.

### Step 5: chiral resolution of methyl dibenzyl[(5-(1-cyclopropylethyl)-7-fluoro-3-methyl-2,3-dihydro-1-benzofuran-4-yl)oxy]phosphate

**Chiral intermediate A1** (12.2 g, retention time 5.976 min) and **chiral intermediate A2** (10.3 g, retention time 9.428 min) were obtained by chiral HPLC resolution. Resolution conditions: instrument: Shamdzu LC20-AP; column: DAICEL CHIRALPAK^{®}IG (250 × 25 mm 10 µm); mobile phase: n-hexane/ethanol = 80/20; wavelength: 254 nm; flow rate: 30 mL/min; temperature: RT; time: 25 min.

### Step 6: synthesis of sodium (5-(1-cyclopropyl)-7-fluoro-3-methyl-2,3-dihydrobenzofuran-4-yl)oxymethyl phosphate (optical isomers 10A1 and 10A2)

Water (10 mL) and Pd/C (600 mg) were added to a solution of methyl dibenzyl[(5-(1-cyclopropylethyl)-7-fluoro-3-methyl-2,3-dihydro-1-benzofuran-4-yl)oxy]phosphate chiral **intermediate A1** (2 g, 3.8 mmol) in tetrahydrofuran (20 mL). The mixture was stirred under hydrogen atmosphere at one standard atmosphere pressure for 2 h and filtered through celite to remove the catalyst. The filtrate was treated with an aqueous sodium carbonate (400 mg, 3.8 mmol) solution (5 mL) and distilled under reduced pressure to remove tetrahydrofuran. The remaining aqueous solution was extracted three times with dichloromethane, and the aqueous layer was lyophilized to give the target product of sodium (5-(1-cyclopropyl)-7-fluoro-3-methyl-2,3-dihydrobenzofuran-4-yl)oxymethyl phosphate **optical isomer 10A1** (1.2 g, 91% yield).

¹H NMR (600 MHz, D₂O) δ 7.16 (d, J = 12.0 Hz, 1H), 5.36 - 5.34 (m, 1H), 5.22 - 5.20 (m, 1H), 4.77 - 4.76 (m, 1H), 4.31 - 4.29 (m, 1H), 3.95 - 3.92 (m, 1H), 2.47 - 2.43 (m, 1H), 1.33 (d, J = 7.2 Hz, 3H), 1.18 (d, J = 6.6 Hz, 3H), 1.05 - 0.99 (m, 1H), 0.59 - 0.54 (m, 1H), 0.40 - 0.36 (m, 1H), 0.33 - 0.30 (m, 1H), 0.18 - 0.14 (m, 1H).

Methyl dibenzyl[(5-(1-cyclopropylethyl)-7-fluoro-3-methyl-2,3-dihydro-1-benzofuran-4-yl)oxy]phosphate **chiral intermediate A2** (2 g, 3.8 mmol) was used to give the target product of sodium (5-(1-cyclopropyl)-7-fluoro-3-methyl-2,3-dihydrobenzofuran-4-yl)oxymethyl phosphate **optical isomer 10A2** (1.1 g, 84% yield) following the same operation steps.

¹H NMR (600 MHz, D₂O) δ 7.11 (d, J = 12.0 Hz, 1H), 5.35 - 5.33 (m, 1H), 5.20 - 5.18 (m, 1H), 4.77 - 4.75 (m, 1H), 4.32 - 4.29 (m, 1H), 3.97 - 3.91 (m, 1H), 2.55 - 2.51 (m, 1H), 1.33 (d, J = 6.6 Hz, 3H), 1.18 (d, J = 7.2 Hz, 3H), 0.96 - 0.90 (m, 1H), 0.53 - 0.49 (m, 1H), 0.30 - 0.21 (m, 2H), 0.10 - 0.06 (m, 1H).

### Synthesis scheme B:

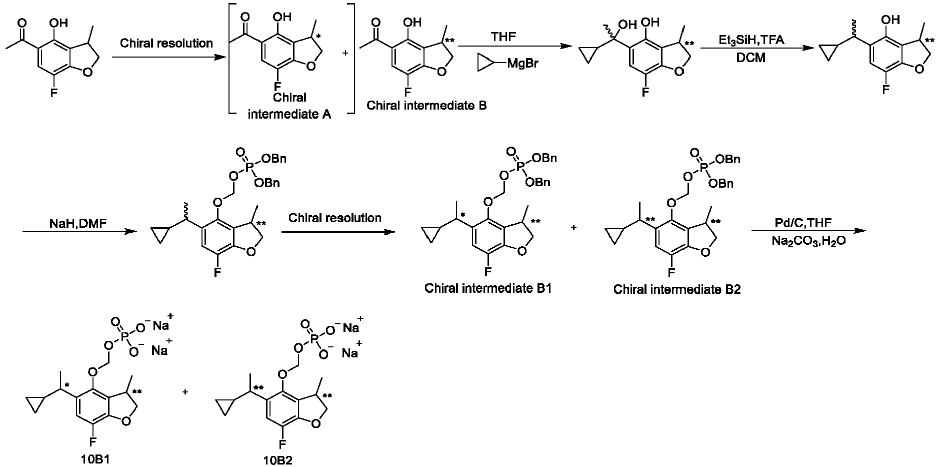

### Step 1: chiral resolution of 1-(7-fluoro-4-hydroxy-3-methyl-2,3-dihydrobenzofuran-5-yl)ethan-1-one

80 g of 1-(7-fluoro-4-hydroxy-3-methyl-2,3-dihydrobenzofuran-5-yl)ethan-1-one was resolved by chiral HPLC to give **chiral intermediate A** (30.5 g, retention time 5.898 min) and chiral **intermediate B** (29.5 g, retention time 6.858 min). Resolution conditions: instrument: Shimadzu LC-20AP; column: DAICEL CHIRALPAK^{®}IA (250 × 25 mm 10 µm); mobile phase: n-hexane/ethanol (0.1% 7 M NH₃ in MeOH) = 80/20; wavelength: 214 nm; flow rate: 30 mL/min; temperature: RT; time: 4.5 min.

### Step 2: synthesis of 5-(1-cyclopropyl-1-hydroxyethyl)-7-fluoro-3-methyl-2,3-dihydrobenzofuran-4-ol

By referring to step 2 of the synthesis method of chiral intermediate A, chiral intermediate B was used as the starting material to synthesize the target product of 5-(1-cyclopropyl-1-hydroxyethyl)-7-fluoro-3-methyl-2,3-dihydrobenzofuran-4-ol (32.6 g).

LC-MS(m/z): [M-H] ⁻ 251.00.

### Step 3: synthesis of 5-(1-cyclopropylethyl)-7-fluoro-3-methyl-2,3-dihydrobenzofuran-4-ol

By referring to step 3 of the synthesis method of chiral intermediate A, 5-(1-cyclopropyl-1-hydroxyethyl)-7-fluoro-3-methyl-2,3-dihydrobenzofuran-4-ol (32.6 g) was used as the starting material to synthesize the target crude product of 5-(1-cyclopropylethyl)-7-fluoro-3-methyl-2,3-dihydrobenzofuran-4-ol (27.5 g).

LC-MS(m/z): [M-H] ⁻ 235.00.

### Step 4: synthesis of methyl dibenzyl[(5-(1-cyclopropylethyl)-7-fluoro-3-methyl-2,3-dihydro-1-benzofuran-4-yl)oxy]phosphate

5-(1-Cyclopropylethyl)-7-fluoro-3-methyl-2,3-dihydrobenzofuran-4-ol (21 g) was dissolved in anhydrous DMF (300 mL). Sodium hydride (4.2 g, 129 mmol) was added under an ice bath. The mixture was stirred for another half an hour under an ice bath. Dibenzylchloromethyl phosphate (23 g, 71 mmol) was added, and the mixture was warmed to room temperature and then stirred overnight. After the reaction was completed, the reaction was quenched with a saturated ammonium chloride solution. The mixture was extracted with ethyl acetate (500 mL), and the organic phase was dried over anhydrous sodium sulfate, concentrated, and purified by a silica gel column (PE/EA = 20%) to give the target product.

LC-MS(m/z): [M+H] ⁺ 527.10.

### Step 5: chiral resolution of methyl dibenzyl[(5-(1-cyclopropylethyl)-7-fluoro-3-methyl-2,3-dihydro-1-benzofuran-4-yl)oxy]phosphate

**Chiral intermediate B1** (10.5 g, retention time 1.956 min) and **chiral intermediate B2** (9.8 g, retention time 2.224 min) were obtained by chiral HPLC resolution. Resolution conditions: instrument: Waters SFC 150; column: DAICEL CHTRALCEL^{®}OJ (250 × 25 mm 10 µm); mobile phase: supercritical CO₂/MeOH (0.1% 7.0 mol/L ammonia in MeOH) = 80/20; wavelength: 214 nm; flow rate: 120 mL/min; temperature: RT; time: 2.5 min.

### Step 6: synthesis of sodium (5-(1-cyclopropyl)-7-fluoro-3-methyl-2,3-dihydrobenzofuran-4-yl)oxymethyl phosphate (optical isomers 10B1 and 10B2)

Water (10 mL) and Pd/C (600 mg) were added to a solution of methyl dibenzyl[(5-(1-cyclopropylethyl)-7-fluoro-3-methyl-2,3-dihydro-1-benzofuran-4-yl)oxy]phosphate **chiral intermediate B1** (2 g, 3.8 mmol) in tetrahydrofuran (20 mL). The mixture was stirred under hydrogen atmosphere at one standard atmosphere pressure for 2 h and filtered through celite to remove the catalyst. The filtrate was treated with an aqueous sodium carbonate (400 mg, 3.8 mmol) solution (5 mL) and distilled under reduced pressure to remove tetrahydrofuran. The remaining aqueous solution was extracted three times with dichloromethane, and the aqueous layer was lyophilized to give the target product of sodium (5-(1-cyclopropyl)-7-fluoro-3-methyl-2,3-dihydrobenzofuran-4-yl)oxymethyl phosphate **optical isomer 10B1** (1.3 g, 99% yield).

¹H NMR (600 MHz, D₂O) δ 7.17 (d, J = 12.0 Hz, 1H), 5.36 - 5.34 (m, 1H), 5.22 - 5.20 (m, 1H), 4.77 - 4.76 (m, 1H), 4.32 - 4.29 (m, 1H), 3.97 - 3.91 (m, 1H), 2.48 - 2.43 (m, 1H), 1.33 (d, J = 6.6 Hz, 3H), 1.18 (d, J = 7.2 Hz, 3H), 1.06 - 1.00 (m, 1H), 0.59 - 0.54 (m, 1H), 0.41 - 0.36 (m, 1H), 0.34 - 0.30 (m, 1H), 0.18 - 0.14 (m, 1H).

Methyl dibenzyl[(5-(1-cyclopropylethyl)-7-fluoro-3-methyl-2,3-dihydro-1-benzofuran-4-yl)oxy]phosphate **chiral intermediate B2** (2 g, 3.8 mmol) was used to give the target product of sodium (5-(1-cyclopropyl)-7-fluoro-3-methyl-2,3-dihydrobenzofuran-4-yl)oxymethyl phosphate **optical isomer 10B2** (1.2 g, 91% yield) following the same operation steps.

¹H NMR (600 MHz, D₂O) δ 7.10 (d, J = 12.0 Hz, 1H), 5.35 - 5.33 (m, 1H), 5.19 - 5.17 (m, 1H), 4.78 - 4.76 (m, 1H), 4.31 - 4.28 (m, 1H), 3.96 - 3.91 (m, 1H), 2.55 - 2.50 (m, 1H), 1.32 (d, J = 6.6 Hz, 3H), 1.27 (d, J = 7.2 Hz, 3H), 0.95 - 0.90 (m, 1H), 0.52 - 0.48 (m, 1H), 0.29 - 0.27 (m, 2H), 0.09 - 0.05 (m, 1H).

### Example 11 Four optical isomers of 5-(1-cyclopropylethyl)-7-fluoro-3-methyl-2,3-dihydrobenzofuran-4-ol (11A1, 11A2, 11B1, and 11B2)

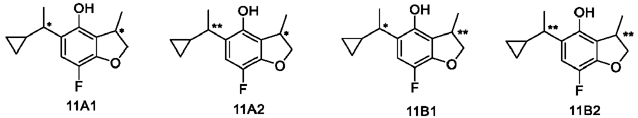

### Synthesis scheme:

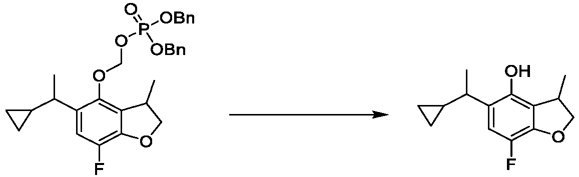

Methyl dibenzyl[(5-(1-cyclopropylethyl)-7-fluoro-3-methyl-2,3-dihydro-1-benzofuran-4-yl)oxy]phosphate **chiral intermediate A1** (1 g, 1.9 mmol) was dissolved in DMF (12 mL). 6 N HCl (8 mL) was added. The mixture was stirred at room temperature overnight, extracted twice with ethyl acetate (50 mL), dried, concentrated, and purified by a column (PE/EA = 8%-10%) to give the target product of 5-(1-cyclopropylethyl)-7-fluoro-3-methyl-2,3-dihydrobenzofuran-4-ol optical isomer **11A1** (310 mg, 69% yield).

¹H NMR (600 MHz, CDCl₃) δ 6.81 (d, J = 12.0 Hz, 1H), 4.72 - 4.70 (m, 2H), 4.26 - 4.23 (m, 1H), 3.65 - 3.59 (m, 1H), 2.40 - 2.35 (m, 1H), 1.37 (d, J = 7.2 Hz, 3H), 1.26 (d, J = 6.6 Hz, 3H), 0.99 - 0.93 (m, 1H), 0.59 - 0.54 (m, 1H), 0.49 - 0.45 (m, 1H), 0.23 - 0.19 (m, 1H), 0.16 - 0.12 (m, 1H).

The same synthesis method was used to obtain 3 other optical isomer products:
Methyl dibenzyl[(5-(1-cyclopropylethyl)-7-fluoro-3-methyl-2,3-dihydro-1-benzofuran-4-yl)oxy]phosphate **chiral intermediate A2** (1 g, 1.9 mmol) was used to synthesize the target product of 5-(1-cyclopropylethyl)-7-fluoro-3-methyl-2,3-dihydrobenzofuran-4-ol optical isomer **11A2** (230 mg, 51% yield)

¹H NMR (600 MHz, CDCl₃) δ 6.82 (d, J = 11.4 Hz, 1H), 4.72 - 4.69 (m, 2H), 4.26 - 4.24 (m, 1H), 3.64 - 3.58 (m, 1H), 2.39 - 2.34 (m, 1H), 1.37 (d, J = 6.6 Hz, 3H), 1.25 (d, J = 7.2 Hz, 3H), 1.01 - 0.95 (m, 1H), 0.59 - 0.55 (m, 1H), 0.51 - 0.46 (m, 1H), 0.23 - 0.19 (m, 1H), 0.17 - 0.12 (m, 1H).

Methyl dibenzyl[(5-(1-cyclopropylethyl)-7-fluoro-3-methyl-2,3-dihydro-1-benzofuran-4-yl)oxy]phosphate **chiral intermediate B1** (800 mg, 1.5 mmol) was used to give the target product of 5-(1-cyclopropylethyl)-7-fluoro-3-methyl-2,3-dihydrobenzofuran-4-ol optical isomer **11B1** (106 mg, 30% yield).

¹H NMR (600 MHz, CDCl₃) δ 6.81 (d, J = 12.0 Hz, 1H), 4.72 - 4.70 (m, 2H), 4.26 - 4.23 (m, 1H), 3.64 - 3.59 (m, 1H), 2.40 - 2.35 (m, 1H), 1.36 (d, J = 6.6 Hz, 3H), 1.26 (d, J = 6.6 Hz, 3H), 0.99 - 0.93 (m, 1H), 0.59 - 0.54 (m, 1H), 0.49 - 0.45 (m, 1H), 0.23 - 0.19 (m, 1H), 0.16 - 0.12 (m, 1H).

Methyl dibenzyl[(5-(1-cyclopropylethyl)-7-fluoro-3-methyl-2,3-dihydro-1-benzofuran-4-yl)oxy]phosphate **chiral intermediate B2** (800 mg, 1.5 mmol) was used to give the target product of 5-(1-cyclopropylethyl)-7-fluoro-3-methyl-2,3-dihydrobenzofuran-4-ol optical isomer **11B2** (200 mg, 56% yield).

¹H NMR (600 MHz, CDCl₃) δ 6.82 (d, J = 11.4 Hz, 1H), 4.72 - 4.66 (m, 2H), 4.26 - 4.23 (m, 1H), 3.64 - 3.58 (m, 1H), 2.39 - 2.34 (m, 1H), 1.37 (d, J = 7.2 Hz, 3H), 1.25 (d, J = 7.2 Hz, 3H), 1.01 - 0.95 (m, 1H), 0.60 - 0.55 (m, 1H), 0.51 - 0.46 (m, 1H), 0.23 - 0.19 (m, 1H), 0.17 - 0.13 (m, 1H).

### Example 12 5-(1-cyclopropylethyl)-7-fluoro-2,3-dihydrobenzofuran-4-ol

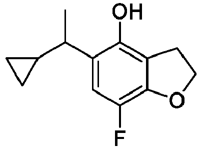

For the synthesis method of Example 12, reference was made to Example 4.

LC-MS: MS Found: 221 [M-H]⁻.

### Example 13 3-cyclopropyl-7-fluoro-5-isopropyl-2,3-dihydrobenzofuran-4-ol

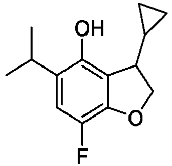

For the synthesis method of Example 13, reference was made to Example 4.

LC-MS: MS Found: 235 [M-H]⁻.

### Example 14 7-fluoro-5-isopropyl-2,3-dimethyl-2,3-dihydrobenzofuran-4-ol

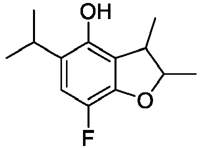

For the synthesis method of Example 14, reference was made to Example 4.

LC-MS: MS Found: 223 [M-H]⁻.

### Example 15 5-(1-cyclopropylethyl)-7-fluoro-2,3-dimethyl-2,3-dihydrobenzofuran-4-ol

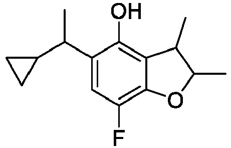

For the synthesis method of Example 15, reference was made to Example 4.

LC-MS: MS Found: 249 [M-H]⁻.

### Biological Test Evaluation

The present invention is further described below using test examples, but these examples are not intended to limit the scope of the present invention.

### Test Example 1: Mouse Righting Reflex Test and LD₅₀ Test

### 1. Objective:

The ED₅₀ value and LD₅₀ value of the compound of the present invention were measured using a sequential method. The TI (therapeutic index) of the drug was calculated. The animal symptoms in the experimental process were observed, and the drug effect, safety and side effects of the compound of the present invention were examined.

### 2. Test animals and administration:

SPF grade ICR mice (Pizhou Oriental Breeding Co., Ltd.), 22-28 g, 15 mice/group. All mice received 3 days of quarantine and acclimation and were transferred from the feeding room to the laboratory for acclimation 1 h prior to the experiment.

The test compound was dissolved in 20% fat emulsion or purified water to prepare a 5 mg/mL drug liquid.

### 3. Test method:

During the test, firstly a mouse was dosed at the tail vein, and the bolus injection was completed within 10 seconds. Whether the mouse was dead after administration was observed, and the righting reflex latency period, the duration and the condition of the mouse after administration were recorded; when the compound did not exert an anesthetic effect (ED₅₀ test) or did not cause death (LD₅₀ test) at the first dose, the next mouse was injected with the drug at the previous dose (that is, 1.25-fold the first dose); when an anesthetic effect or death happened at the first dose of the compound, the next mouse was injected with the next concentration (that is, 0.8-fold the first dose) of the drug; and so on until 6 replicates or 15 mice were completed to terminate the test. Finally, the AOT425 Statpm software was used to calculate the ED₅₀ (median effective dose: the dose required to cause 50% of the mice to lose the righting reflex by the test) and LD₅₀ (median lethal dose: the dose required to cause 50% of the mice to die by the test) values.

The formula for TI (therapeutic index) was: TI = LD₅₀ / ED₅₀.

### 4. Test results: The results are shown in Table 1.

**Table 1. ED₅₀ and LD₅₀ values for the compound of the present invention**

| Example No. | ED₅₀ (mg/Kg) | LD₅₀ (mg/Kg) | TI | Symptoms in test animals in ED₅₀ test |
|---|---|---|---|---|
| Propofol | 12.9 | 55.9 | 4.3 | Some animals exhibited body twitching and hind limb scratching after administration |
| Fospropofol sodium | 111.8 | - | - | Some animals scratched their heads with their hind limbs, had stiff forelimbs, and had an increased heart rate after administration |
| Example 4 | 8.9 | 62.5 | 7.0 | The animals' condition was stable after administration |
| Example 11A1 | 4.0 | - | - | The animals' condition was stable after administration |
| Example 10A1 | 33.5 | - | - | The animals' condition was stable after administration |

### 5. Test conclusion:

From the above data, it can be seen that the compounds of the present invention have a relatively good anesthetic effect, and are safer, with a larger safety window and fewer side effects.

## Claims

1. A compound of general formula (Ia), a stereoisomer thereof, or a pharmaceutically acceptable salt thereof: 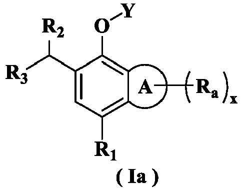 wherein:
ring A is C₅₋₆ cycloalkyl or 5- to 6-membered heterocyclyl;
Rₐ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl;
R₁ is hydrogen, deuterium, halogen, hydroxy, cyano, amino, C₁₋₆ alkoxy, C₁₋₆ alkyl, C₁₋₆ deuterated alkyl, or C₁₋₆ haloalkyl;
R₂ and R₃ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl;
Y is hydrogen, sodium, potassium, C₁₋₆ alkyl, or 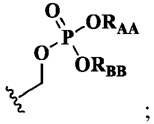
R_{AA} and R_{BB} are each independently hydrogen, deuterium, C₁₋₆ alkyl, C₁₋₆ deuterated alkyl, C₁₋₆ haloalkyl, an alkali metal ion, an alkaline earth metal ion, protonated amine, or protonated amino acid, wherein the alkali metal ion is Na⁺, K⁺, or Li⁺, the alkaline earth metal ion is Be²⁺, Mg²⁺, or Ca²⁺, the protonated amine is tromethamine, triethanolamine, ethanolamine, triethylamine, or N-methylglucamine, and the amino acid is arginine or lysine; and
x is an integer from 0 to 5.

2. A compound of general formula (I), a stereoisomer thereof, or a pharmaceutically acceptable salt thereof: 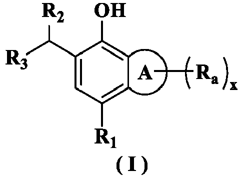 wherein:
ring A is C₅₋₆ cycloalkyl or 5- to 6-membered heterocyclyl;
Rₐ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl;
R₁ is hydrogen, deuterium, halogen, hydroxy, cyano, amino, C₁₋₆ alkoxy, C₁₋₆ alkyl, C₁₋₆ deuterated alkyl, or C₁₋₆ haloalkyl;
R₂ and R₃ are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl; and
x is an integer from 0 to 5.

3. The compound, the stereoisomers thereof, or the pharmaceutically acceptable salt thereof according to claim 1 or 2, wherein one or more of the following conditions are met:
(1) ring A is C₅₋₆ cycloalkyl or 5- to 6-membered heterocyclyl containing 1-3 N, O, or S atoms, preferably 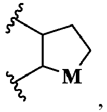 wherein M is CRₐₐR_{bb}, NRₐₐ, O, or S, preferably CRₐₐR_{bb} or O, more preferably CH₂ or O; wherein Rₐₐ and R_{bb} are each independently hydrogen, deuterium, halogen, hydroxy, cyano, amino, C₁₋₆ alkoxy, C₁₋₆ alkyl, C₁₋₆ deuterated alkyl, or C₁₋₆ haloalkyl, preferably hydrogen or C₁₋₆ alkyl, more preferably hydrogen or C₁₋₃ alkyl;
(2) Y is hydrogen or 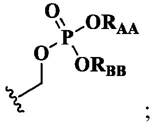
(3) R_{AA} and R_{BB} are each independently hydrogen, C₁₋₆ alkyl, an alkali metal ion, or an alkaline earth metal ion, wherein the alkali metal ion is Na⁺, K⁺, or Li⁺, and the alkaline earth metal ion is Be²⁺, Mg²⁺, or Ca²⁺, preferably the alkali metal ion Na⁺, K⁺, or Li⁺, more preferably Na⁺;
(4) Rₐ are each independently hydrogen, C₁₋₆ alkyl, or C₃₋₆ cycloalkyl, preferably hydrogen, C₁₋₃ alkyl, or C₃₋₅ cycloalkyl, more preferably hydrogen, methyl, ethyl, propyl, or cyclopropyl, further preferably hydrogen, methyl, ethyl, or cyclopropyl;
(5) R₁ is hydrogen or halogen, preferably hydrogen, fluorine, chlorine, or bromine, more preferably hydrogen, fluorine, or chlorine;
(6) R₂ and R₃ are each independently hydrogen, C₁₋₆ alkyl, or C₃₋₆ cycloalkyl, preferably hydrogen, C₁₋₃ alkyl, or C₃₋₅ cycloalkyl, more preferably hydrogen, methyl, ethyl, propyl, or cyclopropyl, further preferably methyl or cyclopropyl;
(7) x is 0, 1, or 2.

4. The compound, the stereoisomer thereof, or the pharmaceutically acceptable salt thereof according to claim 3, wherein

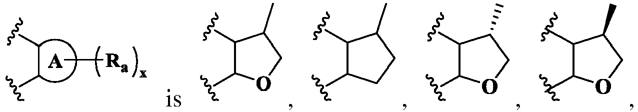

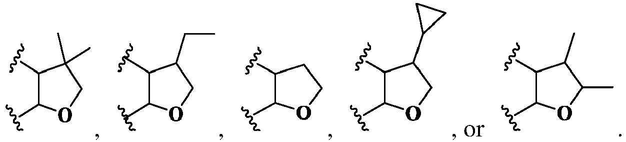

5. The compound of general formula (Ia), the stereoisomer thereof, or the pharmaceutically acceptable salt thereof according to any one of claims 1 and 3-4, wherein general formula (Ia) is further represented by general formula (IIa): 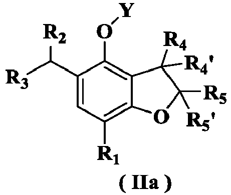 wherein:
R₄, R₄', R₅, and R₅' are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl, preferably hydrogen, C₁₋₆ alkyl, or C₃₋₆ cycloalkyl, more preferably hydrogen, C₁₋₃ alkyl, or C₃₋₅ cycloalkyl, further preferably hydrogen, methyl, ethyl, propyl, or cyclopropyl;
R₁, R₂, R₃, and Y are as described in any one of claims 1 and 3-4.

6. The compound of general formula (I), the stereoisomer thereof, or the pharmaceutically acceptable salt thereof according to any one of claims 2 to 4, wherein general formula (I) is further represented by general formula (II): 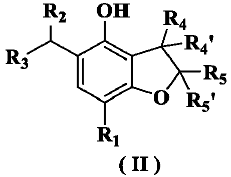 wherein:
R₄, R₄', R₅, and R₅' are each independently hydrogen, deuterium, halogen, hydroxy, cyano, amino, C₁₋₆ alkoxy, C₁₋₆ alkyl, C₁₋₆ deuterated alkyl, C₁₋₆ haloalkyl, C₃₋₆ cycloalkyl, or 3- to 6-membered heterocyclyl, preferably hydrogen, C₁₋₆ alkyl, or C₃₋₆ cycloalkyl, more preferably hydrogen, C₁₋₃ alkyl, or C₃₋₅ cycloalkyl, further preferably hydrogen, methyl, ethyl, propyl, or cyclopropyl;
R₁, R₂, and R₃ are as described in any one of claims 2-4.

7. The compound, the stereoisomers thereof, or the pharmaceutically acceptable salt thereof according to claim 5 or 6, wherein one or more of the following conditions are met:
(1) R₄ and R₄' are each independently hydrogen, methyl, ethyl, or cyclopropyl;
(2) R₅ and R₅' are each independently hydrogen or methyl.

8. The compound, the stereoisomer thereof, or the pharmaceutically acceptable salt thereof according to claim 1, wherein the compound is any one of the following structures: 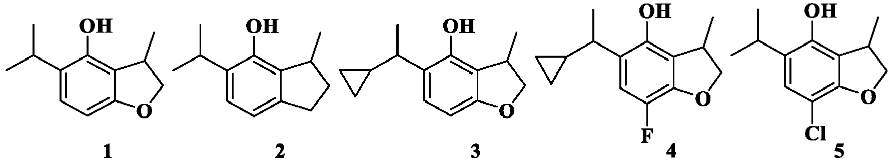 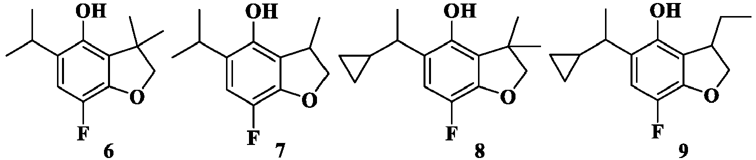 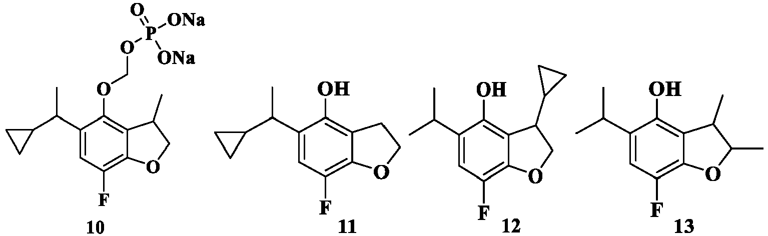 or

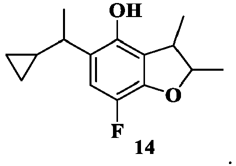

9. The compound, the stereoisomer thereof, or the pharmaceutically acceptable salt thereof according to claim 1, wherein the compound is any one of the following structures: 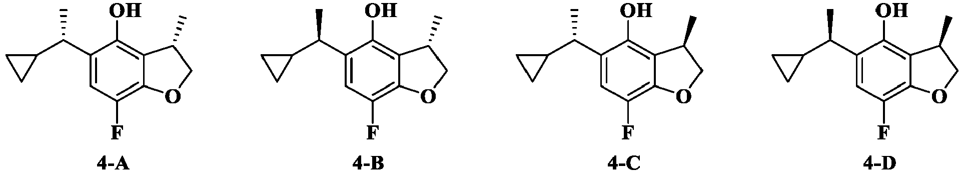 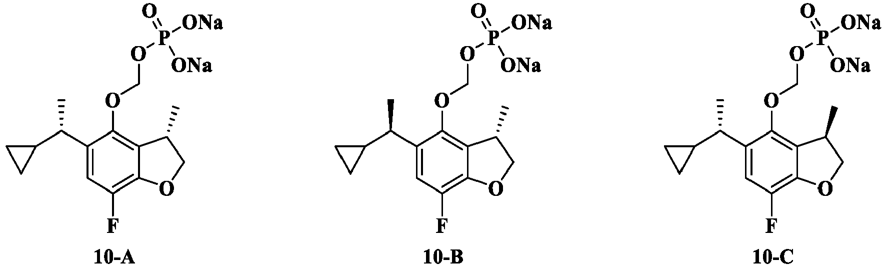 or

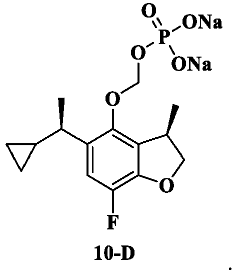

10. A preparation method for a compound of general formula (Ia), a stereoisomer thereof, or a pharmaceutically acceptable salt thereof, wherein the preparation method is any one of the following methods:
method I comprises the following step: in a solvent, performing a dehydroxylation reaction on a compound represented by formula Ia-1 in the presence of a dehydroxylating reagent and an acid to give a compound represented by formula Ia; 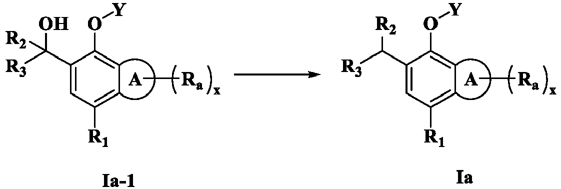
wherein ring A, R₁, R₂ and R₃, Rₐ, Y, and x are as defined in any one of claims 1 and 3-4;
method II comprises the following step: in a solvent, performing a substitution reaction on a compound represented by formula I in the presence of X₂-Y and an alkali to give a compound represented by formula Ia; 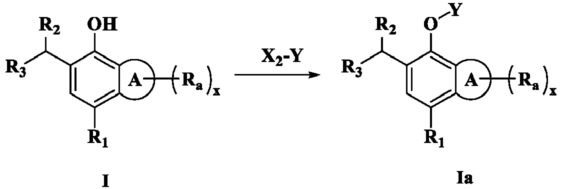
wherein ring A, R₁, R₂ and R₃, Rₐ, Y, and x are as defined in any one of claims 1 and 3-4, and the definition of Y does not include H; X₂ is halogen.

11. A preparation method for a compound of general formula (I), a stereoisomer thereof, or a pharmaceutically acceptable salt thereof, wherein the preparation method comprises the following step: in a solvent, performing a dehydroxylation reaction on a compound represented by formula I-1 in the presence of a dehydroxylating reagent and an acid to give a compound represented by formula I; 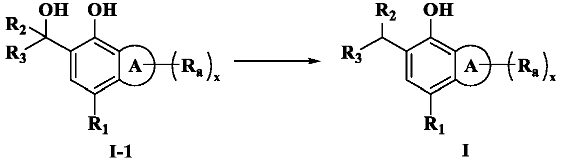 wherein ring A, R₁, R₂ and R₃, Rₐ, and x are as defined in any one of claims 2-4.

12. A pharmaceutical composition comprising a therapeutically effective dose of the compound, the stereoisomer thereof, or the pharmaceutically acceptable salt thereof according to any one of claims 1 to 9, and one or more pharmaceutically acceptable carriers or excipients.

13. Use of the compound, the stereoisomer thereof, or the pharmaceutically acceptable salt thereof according to any one of claims 1-9, or the pharmaceutical composition according to claim 12 in the preparation of a GABA_{A} receptor agonist drug.

14. Use of the compound, the stereoisomer thereof, or the pharmaceutically acceptable salt thereof according to any one of claims 1-9, or the pharmaceutical composition according to claim 12 in the preparation of a drug in the central nervous system field.

15. The use according to claim 14, wherein the drug in the central nervous system field is a drug for inducing and maintaining anesthesia in a mammal, promoting sedation and hypnosis in a mammal, and treating and/or preventing anxiety, depression, insomnia, nausea, vomiting, migraine, schizophrenia, convulsions, or epilepsy.
